# EUROPEAN PATENT SPECIFICATION

(11) **EP 4 394 379 B1**
(45) Date of publication and mention of the grant of the patent: **12.08.2026**
(21) Application number: 23219232.8
(22) Date of filing: 21.12.2023
(51) Int. Cl.: G01N 33/49, G01N 33/86

(54) **METHOD FOR DETERMINING BAD COLLECTION OF BLOOD, BLOOD COAGULATION ANALYSIS METHOD, AND BLOOD COAGULATION ANALYSIS SYSTEM**
VERFAHREN ZUR BESTIMMUNG SCHLECHTER BLUTABNAHME, BLUTGERINNUNGSANALYSEVERFAHREN UND BLUTGERINNUNGSANALYSESYSTEM
PROCÉDÉ DE DÉTERMINATION D'UNE PRISE MAUVAISE DE SANG, PROCÉDÉ D'ANALYSE DE COAGULATION SANGUINE ET SYSTÈME D'ANALYSE DE COAGULATION SANGUINE

(30) Priority: 26.12.2022 JP 2022208700
(43) Date of publication of application: 03.07.2024
(73) Proprietor: SYSMEX CORPORATION, Kobe-shi Hyogo 651-0073 (JP)
(72) Inventor: Nishi, Keisuke, Kobe-shi, Hyogo 651-0073 (JP); Tabuchi, Yuka, Kobe-shi, Hyogo 651-0073 (JP); Suzuki, Takeshi, Kobe-shi Hyogo 651-0073 (JP)
(74) Representative: Hoffmann Eitle

(56) References cited:
- US-A1- 2022 115 091
- G. LIPPI ET AL: "Right or wrong sample received for coagulation testing? Tentative algorithms for detection of an incorrect type of sample", INTERNATIONAL JOURNAL OF LABORATORY HEMATOLOGY, vol. 32, no. 1p2, 9 February 2009 (2009-02-09), GB; US, pages 132 - 138, XP055268189, ISSN: 1751-5521, DOI: 10.1111/j.1751-553X.2009.01142.x
- A. MAGNETTE: "Pre-analytical issues in the haemostasis laboratory: guidance for the clinical laboratories", THROMBOSIS JOURNAL, vol. 14, no. 1, 12 December 2016 (2016-12-12), GB, XP093159592, ISSN: 1477-9560, DOI: 10.1186/s12959-016-0123-z

## Description

### CROSS REFERENCE TO RELATED APPLICATIONS

This application claims priority from prior Japanese Patent Application No. 2022-208700, filed on December 26, 2022, entitled "POOR BLOOD COLLECTION DETERMINATION METHOD, BLOOD COAGULATION ANALYSIS METHOD, COMPUTER PROGRAM, AND BLOOD COAGULATION ANALYSIS SYSTEM".

### FIELD OF THE INVENTION

The present invention relates to a poor blood collection determination method for performing determination as to poor blood collection of a blood specimen, a blood coagulation analysis method, and a blood coagulation analysis system.

### BACKGROUND OF THE INVENTION

In a blood coagulation test, a tissue fluid may be mixed in a syringe depending on a blood collection technique. If a tissue fluid containing a tissue factor is mixed in a blood specimen, blood coagulation is initiated and fibrin deposition is started, so that the test result may not represent a pathological condition of a patient. Therefore, in a case where blood collection is determined to be poor, blood needs to be collected again ("Kessen shiketsu no rinshou - kennshuui no tameni V 8. Rinshou kensashitsu kara rinshou he (deta to tomoni jyouhou wo) (Clinical thrombus and hemostasis - for residents V 8. Data with information from laboratory to clinician)" written by Yutaka KOMIYAMA, The Japanese Society on Thrombosis and Hemostasis, September 9, 2008, The Japanese Journal of Thrombosis and Hemostasis, Volume 19, No. 4, p.474-477).

US 2022/115091 A1 discloses a deep learning system for assessing blood collection quality based on the coagulation curve and its derivatives.

The poor blood collection is determined by visually observing a specimen. However, the determination accuracy may vary depending on proficiency of a determiner. Therefore, a method for objectively detecting poor blood collection is required.

### SUMMARY OF THE INVENTION

A poor blood collection determination method of the present invention includes: mixing a blood specimen and a blood coagulation measurement reagent to prepare a measurement sample; obtaining measurement values representing a degree of coagulation of the blood specimen from the measurement sample; obtaining coagulation waveform data of the blood specimen based on a plurality of the measurement values; and performing determination as to poor blood collection of the blood specimen based on the coagulation waveform data, wherein the performing the determination comprises obtaining values of a plurality of kinds of parameters concerning coagulation of the blood specimen based on the coagulation waveform data, and performing determination as to poor blood collection of the blood specimen based on the obtained values of the plurality of kinds of parameters, wherein the plurality of kinds of parameters include one or more kinds of parameters concerning a coagulation rate of a blood specimen and one or more kinds of parameters concerning a coagulation time of a blood specimen.

A blood coagulation analysis method of the present invention includes steps of the above-described poor blood collection determination method of the present invention; and obtaining an analytic value concerning coagulability of the blood specimen based on the coagulation waveform data.

A method of the present invention which is performed by a computer for performing determination as to poor blood collection of a blood specimen includes: receiving coagulation waveform data, of a blood specimen, which is based on measurement values that represent a degree of coagulation of the blood specimen and that are obtained from a measurement sample containing the blood specimen and a blood coagulation measurement reagent; and performing determination as to poor blood collection of the blood specimen based on the coagulation waveform data, wherein values of a plurality of kinds of parameters concerning coagulation of a blood specimen are obtained on the basis of coagulation waveform data based on the measurement values representing a degree of coagulation, and determination as to poor blood collection of the blood specimen is performed based on the obtained values of the plurality of kinds of parameters, wherein the plurality of kinds of parameters include one or more kinds of parameters concerning a coagulation rate of a blood specimen and one or more kinds of parameters concerning a coagulation time of a blood specimen.

A method of the present invention which is performed by a computer for performing determination as to poor blood collection of a blood specimen includes: receiving values of a plurality of kinds of parameters concerning coagulation of a blood specimen, the values of the plurality of kinds of parameters being determined from coagulation waveform data, of the blood specimen, which is based on measurement values that represent a degree of coagulation of the blood specimen and that are obtained from a measurement sample containing the blood specimen and a blood coagulation measurement reagent; and performing determination as to poor blood collection of the blood specimen based on the values of the plurality of kinds of parameters, wherein the plurality of kinds of parameters include one or more kinds of parameters concerning a coagulation rate of a blood specimen and one or more kinds of parameters concerning a coagulation time of a blood specimen.

A blood coagulation analysis system (1000, 2000) of the present invention includes: a sample preparation unit (23) configured to mix a blood specimen and a blood coagulation measurement reagent to prepare a measurement sample; a detector (22) configured to obtain measurement values representing a degree of coagulation of the blood specimen, from the measurement sample; and a controller (10, 24, 101, 102) configured to generate coagulation waveform data of the blood specimen based on the measurement values, obtain an analytic value concerning coagulability of the blood specimen based on the coagulation waveform data, and perform determination as to poor blood collection of the blood specimen based on the coagulation waveform data, wherein values of a plurality of kinds of parameters concerning coagulation of a blood specimen are obtained on the basis of coagulation waveform data based on the measurement values representing a degree of coagulation, and determination as to poor blood collection of the blood specimen is performed based on the obtained values of the plurality of kinds of parameters, wherein the plurality of kinds of parameters include one or more kinds of parameters concerning a coagulation rate of a blood specimen and one or more kinds of parameters concerning a coagulation time of a blood specimen.

The present invention can provide a method for objectively detecting poor blood collection without depending on the subjective point of view of a determiner.

### BRIEF DESCRIPTION OF THE DRAWINGS

FIG. 1 illustrates an outer appearance of an analysis system 1000;
FIG. 2 illustrates a functional configuration of the analysis system 1000;
FIG. 3 illustrates information stored in a storage unit 13;
FIG. 4 illustrates a configuration of a light application unit 20;
FIG. 5A illustrates a configuration of a detector 22 in which a cuvette 80 is not set;
FIG. 5B illustrates a configuration of the detector 22 in which the cuvette 80 is set;
FIG. 6 is a plan view of a sample preparation unit 23 as viewed from above;
FIG. 7 is a flow chart showing processes performed by a controller 10 and a controller 24;
FIG. 8 is a flow chart showing a measurement process performed by the controller 24;
FIG. 9 is a flow chart showing a poor blood collection determination process performed by the controller 10;
FIG. 10A illustrates an example of coagulation waveform data;
FIG. 10B illustrates an example of a coagulation waveform;
FIG. 10C illustrates an example of a normalized coagulation waveform;
FIG. 11 illustrates an example of a first-order differential curve of a coagulation waveform;
FIG. 12 illustrates an example of a scattergram of coagulation times and coagulation rates;
FIG. 13A illustrates an example of a coagulation waveform of a poorly collected blood specimen;
FIG. 13B illustrates an example of a coagulation waveform of a normal specimen;
FIG. 14A illustrates an example of a scattergram including a demarcation line between poorly collected blood specimens and normal specimens;
FIG. 14B shows a table indicating an example of test data in the poor blood collection determination process;
FIG. 14C illustrates an example of an ROC curve for evaluating fractionation performance of a learning model;
FIG. 15A illustrates an example of an analysis result output screen;
FIG. 15B illustrates an example of an analysis result output screen;
FIG. 15C illustrates an example of an analysis result output screen;
FIG. 15D illustrates an example of an analysis result output screen;
FIG. 16 illustrates a coagulation waveform to which fitting of a mathematical model has been performed;
FIG. 17 shows a table indicating an example of test data in the poor blood collection determination process;
FIG. 18 illustrates an example of a second-order differential curve of a coagulation waveform;
FIGS. 19A to 19I illustrate examples of scattergrams based on different kinds of parameters;
FIG. 20 shows a table indicating an example of test data in the poor blood collection determination process;
FIG. 21 illustrates an outer appearance of an analysis system 2000;
FIG. 22 is a flow chart showing processes performed by the controller 10, the controller 24, and a controller 30;
FIG. 23 illustrates an example of coagulation waveform data;
FIG. 24 schematically illustrates learning of a deep learning algorithm;
FIG. 25 is a flow chart showing a poor blood collection determination process performed by the controller 10 or the controller 30;
FIG. 26 schematically illustrates analysis performed by a deep learning algorithm;
FIG. 27 shows a table indicating an example of test data in the poor blood collection determination process; and
FIG. 28 illustrates an example of an ROC curve for evaluating fractionation performance of the deep learning algorithm.

### DETAILED DESCRIPTION

### (First embodiment)

### 1. Analysis system 1000

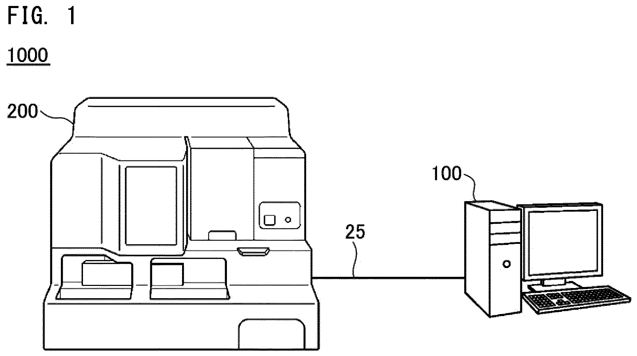

A blood coagulation analysis system 1000 (hereinafter, referred to as analysis system 1000) according to the present embodiment will be described with reference to FIG. 1. The analysis system 1000 includes an analyzer 100 for analyzing coagulability of a blood specimen, and a measurement unit 200 for applying light to a blood specimen, obtaining optical information, and obtaining coagulation waveform data. The analyzer 100 and the measurement unit 200 are connected to each other via a communication cable 25. The analyzer 100 and the measurement unit 200 may be integrated with each other as one device.

Examples of a blood specimen to be analyzed by the analysis system 1000 include whole blood and plasma. The blood specimen is preferably plasma. When blood is collected, an anticoagulant agent other than heparin, warfarin, and direct oral anticoagulant (DOAC) may be added to the blood specimen. As such an anticoagulant agent, citrates such as trisodium citrate can be used. A subject is not particularly limited, and is, for example, a subject whose pathological condition needs to be, for example, grasped by using a coagulation test. A kind of a measurement item concerning coagulability for which the coagulation test is to be performed is not particularly limited, and is, for example, a coagulation time such as an activated partial thromboplastin time (APTT), a prothrombin time (PT), and a thrombin time (TT).

### 1.1 Analyzer 100

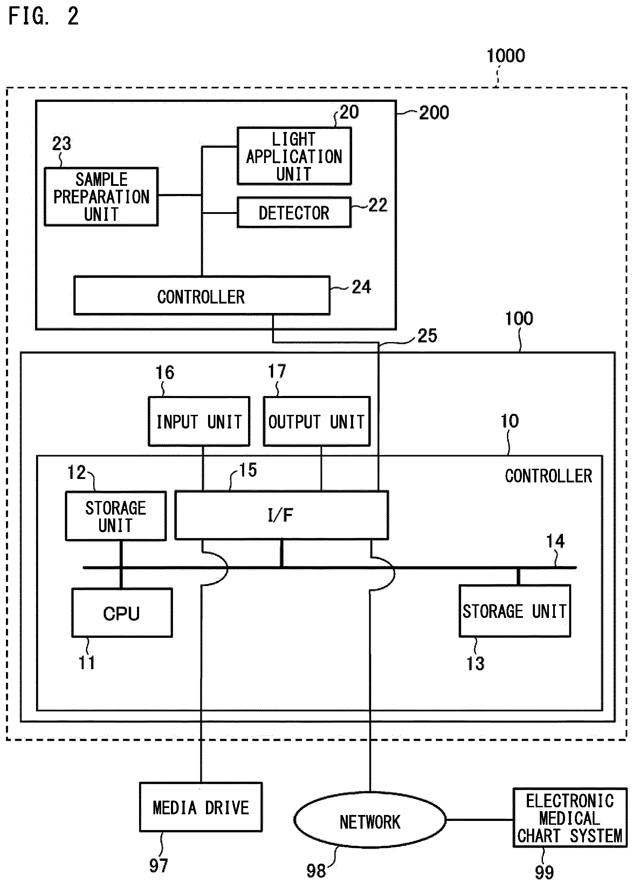

The analyzer 100 according to the present embodiment will be described with reference to FIG. 2. The analyzer 100 is connected to the measurement unit 200 so as to be able to communicate therewith via the communication cable 25. The analyzer 100 is connected to a media drive 97. The analyzer 100 is connected to an electronic medical chart system 99 via a network 98. The network 98 is, for example, a local area network (LAN). The network 98 may be the Internet.

The analyzer 100 includes a controller 10, an input unit 16, and an output unit 17. The controller 10 includes a CPU (central processing unit) 11 that performs data processing, a storage unit 12 used as a work area for the data processing, a storage unit 13 that stores information to be transferred to the storage unit 12, a bus 14 via which data is transmitted between devices, and an interface (I/F) 15 for performing data input from and data output to an external device. The input unit 16, the output unit 17, the media drive 97, the network 98, and the measurement unit 200 are connected to the interface 15.

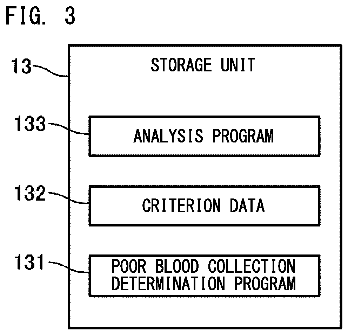

The storage unit 12 is implemented by a DRAM and a SRAM. The storage unit 13 is a solid-state drive. The storage unit 13 may be a hard disk drive. The interface 15 is Ethernet (Registered Trademark). The interface 15 may be IEEE1394, a USB, or the like. The input unit 16 is implemented by a keyboard and a mouse. The output unit 17 is implemented by a liquid crystal display. The output unit 17 may be an organic EL display. A determination result concerning poor blood collection of a blood specimen and an analytic value concerning coagulability are outputted to the output unit 17. As shown in FIG. 3, the storage unit 13 stores a poor blood collection determination program 131 for determining whether or not blood collection of a blood specimen to be tested is poor, criterion data 132 used as a criterion for poor blood collection, and an analysis program 133 for analyzing coagulability of a blood specimen to be tested.

### 1.2 Configuration of measurement unit

The measurement unit 200 will be described with reference to FIG. 2. The measurement unit 200 includes a light application unit 20, a detector 22, a sample preparation unit 23, and a controller 24.

The controller 24 controls the light application unit 20, the detector 22, and the sample preparation unit 23, and performs data communication with the controller 10 of the analyzer 100. Similarly to the controller 10, the controller 24 includes a CPU, a DRAM, a SRAM, a solid-state drive, and Ethernet. In the solid-state drive, a measurement program performed by the measurement unit 200 for measuring blood coagulation is stored.

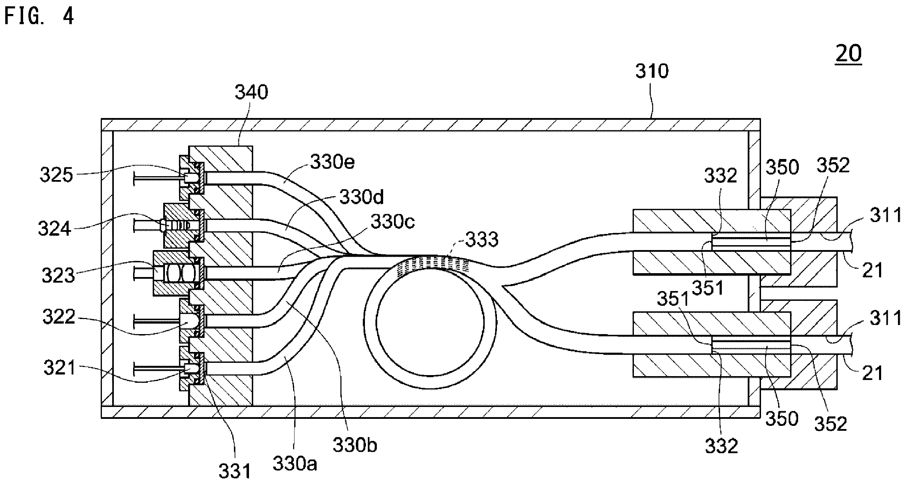

The light application unit 20 according to the present embodiment will be described with reference to FIG. 4. The light application unit 20 includes five light sources 321, 322, 323, 324, and 325, five optical fiber parts 330a, 330b, 330c, 330d, and 330e, and one holding member 340. The optical fiber parts 330a to 330e are disposed so as to correspond to the five light sources 321 to 325, respectively. The holding member 340 holds the light sources 321 to 325, and incident ends 331 of the optical fiber parts 330a to 330e. The light sources 321 to 325, the optical fiber parts 330a to 330e, and the holding member 340 are housed in a metal housing 310. The light sources 321 to 325 are each implemented by a LED. The first light source 321 emits 660 nm light, the second light source 322 emits 405 nm light, the third light source 323 emits 800 nm light, the fourth light source 324 emits 340 nm light, and the fifth light source 325 emits 575 nm light. The controller 24 controls light emission and light extinguishment of the light sources 321 to 325.

The optical fiber parts 330a to 330e are each implemented by a cable which has one core and bundled optical fiber element wires. The optical fiber parts 330a to 330e are bundled into one at an intermediate part 333. The optical fiber parts 330a to 330e bundled into one are divided into two bundles such that each bundle uniformly includes the optical fiber element wires of the optical fiber parts 330a to 330e, and an emission end 332 of each bundle is held at an outlet 311 disposed in the housing 310. An incident end 352 of an optical fiber 21 is also held at the outlet 311. The optical fiber 21 connects between the light application unit 20 and the detector 22. Light from the light application unit 20 is transmitted through the optical fiber 21 to the detector 22. A homogenizing member 350 is disposed between the emission end 332 of the optical fiber parts 330a to 330e and the incident end 352 of the optical fiber 21. The homogenizing member 350 homogenizes an intensity distribution of light emitted through the emission end 332. The homogenizing member 350 reflects therein light incident on an incident surface 351 multiple times, and is a polygonal-prism-shaped rod homogenizer.

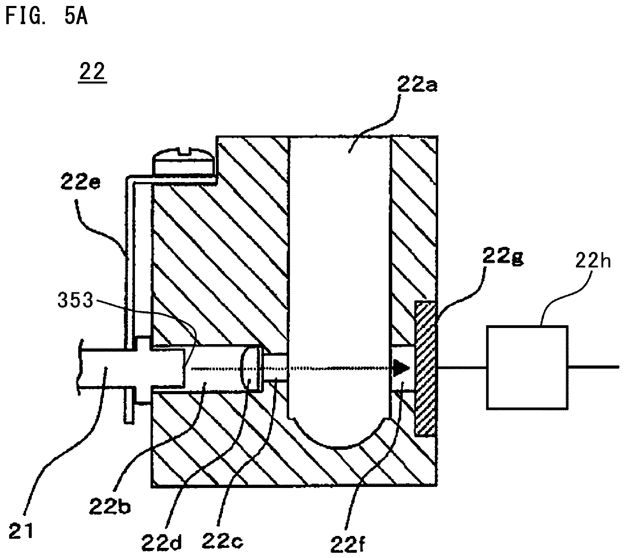

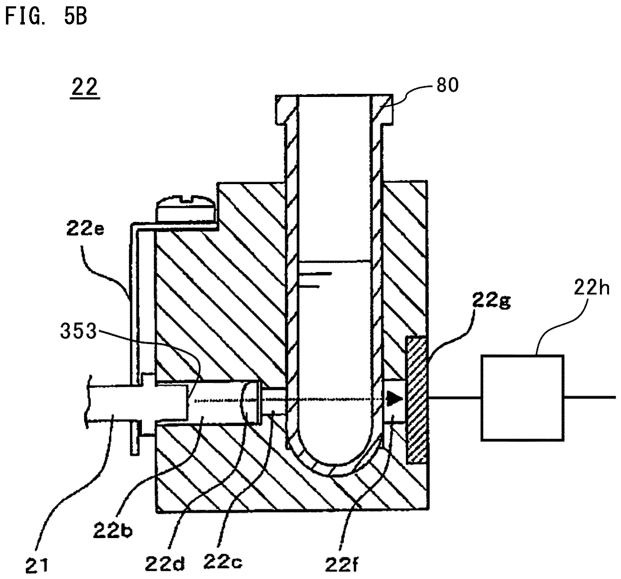

The detector 22 according to the present embodiment will be described with reference to FIGS. 5A and 5B. The detector 22 is disposed at each of the optical fibers 21 connected to the light application unit 20. The structure for connection between the optical fiber 21 and the detector 22 and the hardware configuration of the detector 22 are the same among all the detectors 22. Therefore, one detector 22 will be described here. As shown in FIG. 5A, the detector 22 has a hole 22b in which the other end 353 of the optical fiber 21 is inserted. The incident end 352 of the optical fiber 21 is held at the outlet 311 of the light application unit 20 (see FIG. 4). The detector 22 has a holding part 22a for holding a cuvette 80, the hole 22b, and a connection hole 22c. The connection hole 22c connects the hole 22b to the holding part 22a.

The diameter of the hole 22b is greater than the diameter of the connection hole 22c. A lens 22d for condensing light from the optical fiber 21 is disposed at the end portion of the hole 22b. Furthermore, a hole 22f is formed in an inner wall surface of the holding part 22a so as to oppose the connection hole 22c. A light receiver 22g is disposed deeply in the hole 22f. The light receiver 22g is implemented by a photodiode, and outputs an electric signal corresponding to an amount of received light. Light transmitted through the lens 22d is incident on a light receiving surface of the light receiver 22g through the connection hole 22c, the holding part 22a, and the hole 22f. The optical fiber 21 is fixed by a plate spring 22e in a state where the end portion 353 is inserted in the hole 22b.

FIG. 5B illustrates a state in which the cuvette 80 is held at the holding part 22a, and light condensed by the lens 22d is transmitted through the cuvette 80 and a sample contained in the cuvette 80, and is incident on the light receiver 22g. Progress of a coagulation reaction in the sample causes increase of turbidity of the sample. According thereto, an amount of light (amount of transmitted light) transmitted through the sample is reduced, and the level of the electric signal outputted from the light receiver 22g is lowered. The electric signal outputted from the light receiver 22g is converted to a digital signal by an A/D converter 22h, and transmitted to the controller 24. The signal outputted from the light receiver 22g and converted by the A/D converter 22h is optical information that represents a measurement value indicating an amount of transmitted light. Since the turbidity of the sample is increased and an amount of transmitted light is reduced according to progress of a coagulation reaction in the sample, the amount of transmitted light is a measurement value indicating a degree of coagulation.

In the above-described example, the light receiver 22g of the detector 22 detects the transmitted light. However, the detector 22 may be configured to receive light (scattered light) scattered by a sample contained in the cuvette 80. For example, the light receiver 22g may be disposed in the direction intersecting the direction in which light condensed by the lens 22d propagates. In this case, the controller 10 analyzes a coagulation reaction based on the change of the scattered light intensity. The detector 22 that detects the scattered light has a hole that is located at the same height as the connection hole 22c, in an inner surface of the holding part 22a, and a light detector is disposed deeply in the hole. When the cuvette 80 is held at the holding part 22a and light is applied thereto, light scattered by the sample in the cuvette 80 is applied to the light detector through the hole The detection signal from the light detector represents an intensity of the scattered light from the sample. Since turbidity of the sample is increased and an intensity of the scattered light is enhanced according to progress of a coagulation reaction in the sample, an intensity of the scattered light is a measurement value indicating a degree of coagulation.

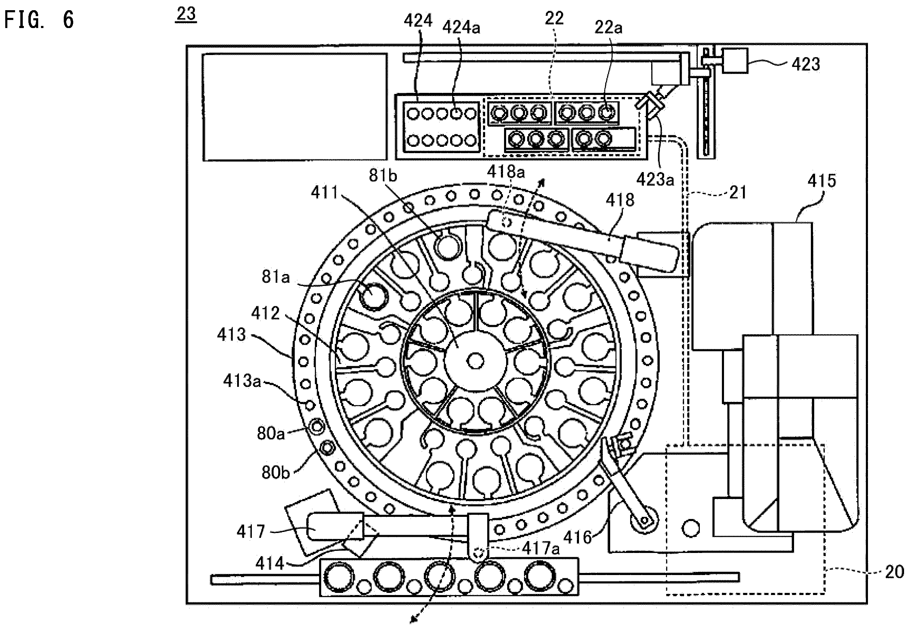

The sample preparation unit 23 according to the present embodiment will be described with reference to FIG. 6. Reagent tables 411 and 412 and a cuvette table 413 each have an annular shape, and are rotatable. The reagent tables 411 and 412 correspond to reagent storage parts, and reagent containers 81a, 81b, etc., are placed on the reagent tables 411 and 412. The number of the reagent containers 81 may be one, or may be three or more. To each of the reagent containers 81a, 81b, etc., a bar code label on which a bar code representing a kind of a reagent contained therein, and a reagent ID as a serial number assigned to the reagent is printed, is adhered. A bar code reader 414 reads the bar code of each of the reagent containers 81a, 81b placed on the reagent tables 411 and 412. The information (kind of the reagent, the reagent ID) read from the bar code is inputted to the controller 10 and stored in the storage unit 13 (see FIG. 2). The reagent container 81a in which the APTT measurement reagent is stored and the reagent container 81b in which a calcium solution is stored are placed on the reagent table 411 and/or the reagent table 412.

The cuvette table 413 has a support part 413a including a plurality of holes in which the cuvettes 80a, 80b, etc., can be supported. The cuvettes 80a, 80b, etc., which are put anew in a cuvette supply part 415 by a user are sequentially transferred by the cuvette supply part 415, and set at the support part 413a of the cuvette table 413 by a cuvette transfer part 416. The cuvettes 80a, 80b, etc., have the same structure.

A stepping motor is connected to each of a specimen dispensing arm 417 and a reagent dispensing arm 418 in order to allow the specimen dispensing arm 417 and the reagent dispensing arm 418 to move upward and downward and rotate. A pipette 417a having a sharp tip is set at the end of the specimen dispensing arm 417 so as to puncture a lid of a specimen container. A pipette 418a is set at the end of the reagent dispensing arm 418. The end of the pipette 418a is formed so as to be flat unlike the pipette 417a.

A heating unit 424 includes a plurality of heating holes 424a, and heats samples contained in the cuvettes 80a, 80b, etc., which are set in the heating holes 424a. A cuvette transfer part 423 includes a catcher 423a for holding the cuvette 80a, 80b, etc., and transfers the cuvette 80a, 80b, etc., at the cuvette table 413, to the heating hole 424a of the heating unit 424 and the holding part 22a of the detector 22.

As the measurement unit 200, the device disclosed in US Patent No. 10,048,249 can be used.

### 1.3 Measurement process and analysis process

A measurement process is performed by the measurement unit 200 under the control of the controller 24, and an analysis process is performed by the analyzer 100 under the control of the controller 10. However, the present disclosure is not limited thereto. For example, the measurement process and the analysis process may be performed under the control of the controller 10, and the measurement process and the analysis process may be performed under the control of the controller 24.

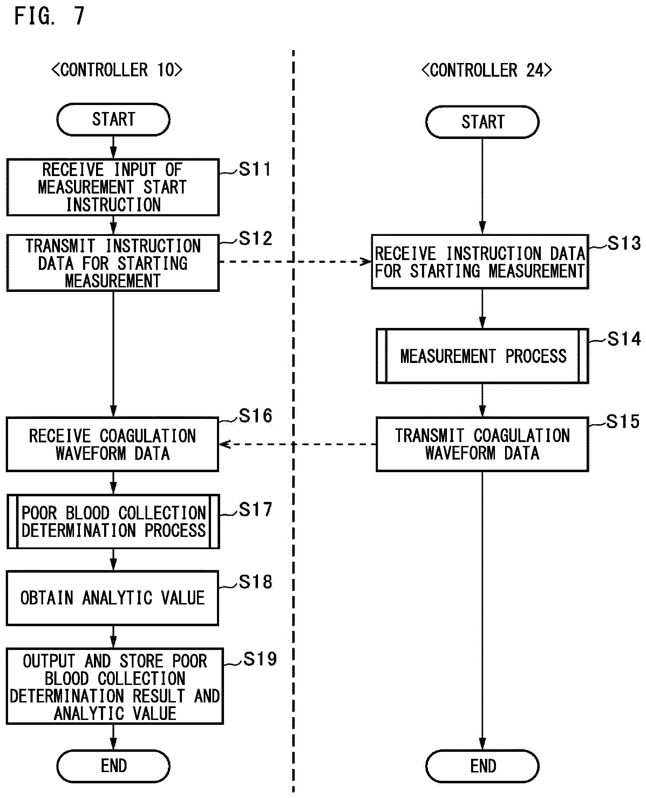

The measurement process and the analysis process performed under the control of the controller 24 and the controller 10 will be described with reference to FIG. 7. In step S11, the controller 10 operates to receive a measurement start instruction inputted by the user through the input unit 16. In step S12, the controller 10 operates to transmit instruction data for starting the measurement to the controller 24 of the measurement unit 200. When the instruction data is received in step S13, the controller 24 operates to perform the measurement process based on the measurement program in step S14. In step S15, the controller 24 operates to transmit, to the controller 10, coagulation waveform data based on measurement values that are obtained in the measurement process, and end the process. The controller 10 operates to receive the coagulation waveform data in step S16, and store the coagulation waveform data in the storage unit 13. The controller 10 performs a process of performing determination as to poor blood collection of the blood specimen in step S17. In step S18, the controller 10 performs a process of obtaining an analytic value concerning coagulability of the blood specimen. In step S19, the controller 10 operates to output a determination result and the analytic value to the output unit 17. Furthermore, the controller 10 operates to store the determination result and the analytic value in the storage unit 13 in step S19.

### 1.4 Measurement process based on measurement program (step S14)

In the measurement process of step S14, a blood specimen and an APTT measurement reagent are mixed to prepare a measurement sample, and a coagulation time of the prepared measurement sample is measured. In the measurement, after the APTT measurement reagent is mixed with the blood specimen and the measurement sample is incubated, a calcium solution is added to the obtained product, measurement values indicating a degree of coagulation are obtained, and coagulation waveform data is obtained based on a plurality of measurement values. For example, the measurement sample is incubated at a constant temperate determined in a range of 30°C or higher and 42°C or lower. The measurement sample is preferably incubated at 37°C.

The APTT measurement reagent refers to a reagent containing an activator and phospholipid. The activator refers to a substance for activating a contact factor in an intrinsic coagulation pathway. Examples of the activator include ellagic acid compounds, silica, kaolin, and Celite. The ellagic acid compound may be any one of ellagic acid, an ellagic acid salt, and a metal complex of ellagic acid. One kind of the activator may be used, or two or more kinds of the activators may be used in combination. The activator is preferably an ellagic acid compound. The ellagic acid compound is particularly preferably a metal complex of ellagic acid which contains metal ions such as zinc ions, manganese ions, or aluminium ions. Examples of the phospholipid include phosphatidylethanolamine (PE), phosphatidylcholine (PC), and phosphatidylserine (PS). The APTT measurement reagent contains one kind of phospholipid selected from PE, PC, and PS, preferably contains two kinds of the phospholipids, and more preferably contains all the kinds of the phospholipids. The phospholipid may be naturally-occurring phospholipid or synthetic phospholipid.

As the APTT measurement reagent, commercially available reagents such as Revohem (Registered Trademark) APTT SLA (SYSMEX CORPORATION), Thrombocheck (Registered Trademark) APTT SLA (SYSMEX CORPORATION), Coagpia (Registered Trademark) APTT-N (SEKISUI MEDICAL CO., LTD.), and Data-Fi·APTT (Siemens Healthcare Diagnostics Products GmbH) may be used.

The calcium solution is a reagent for starting blood coagulation by supplying calcium ions to the mixture of the blood specimen and the APTT measurement reagent. The calcium solution can be an aqueous solution containing calcium ions. The calcium solution is preferably an aqueous solution of a calcium salt such as calcium chloride. The concentration of calcium ions in the calcium solution is, for example, 10 mM or more and 30 mM or less and preferably 20 mM or 25 mM. In a case where a calcium salt such as calcium chloride which is easily dissolved in water is used, the concentration of calcium ions in the calcium solution can be expressed by the concentration of the calcium salt.

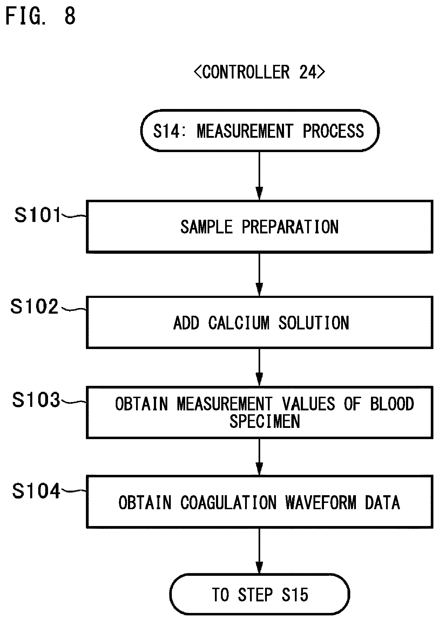

When step S14 shown in FIG. 7 is started, the measurement process shown in FIG. 8 is performed. Step S101 will be described with reference to FIG. 6 and FIG. 8. In step S101, the controller 24 controls the sample preparation unit 23 so as to prepare a measurement sample by mixing a blood specimen and a blood coagulation measurement reagent. According to the control, a blood specimen (plasma) of a subject in a specimen container is suctioned by the pipette 417a of the specimen dispensing arm 417, and the blood specimen is dispensed into an empty cuvette 80a on the cuvette table 413. Subsequently, the cuvette table 413 is rotated, the cuvette transfer part 423 transfers the cuvette 80a containing the blood specimen of the subject into a range in which the pipette 418a of the reagent dispensing arm 418 is movable, and the APTT measurement reagent suctioned from the reagent container 81a is added into the cuvette 80a by the pipette 418a. Thus, a measurement sample is prepared. Thereafter, the cuvette transfer part 423 transfers the cuvette 80a to the heating unit 424, and heats the measurement sample in the cuvette 80a to a predetermined temperature (37°C).

In step S102, the controller 24 controls the sample preparation unit 23 so as to add the calcium solution to the measurement sample and transfer the cuvette to the detector 22. According to the control, the cuvette transfer part 423 transfers the cuvette 80a containing the heated measurement sample from the heating unit 424 into a range in which the pipette 418a is movable. The calcium solution suctioned from the reagent container 81b is added into the cuvette 80a by the pipette 418a. Immediately after the calcium solution is added, the cuvette transfer part 423 transfers the cuvette 80a to which the calcium solution has been added, to the holding part 22a of the detector 22.

Step S103 will be described with reference to FIG. 4, FIG. 5B, FIG. 6, and FIG. 8. In step S103, the controller 24 controls the measurement unit 200 so as to obtain a measurement value representing a degree of coagulation of the blood specimen, from the measurement sample. According to the control, the light application unit 20 applies light to the cuvette 80a which has been transferred to the holding part 22a. In this example, only the first light source 321 applies light. The detector 22 transmits, to the controller 24, digital data corresponding to an intensity of the received light. Specifically, the light receiver 22g outputs analog signals corresponding to an intensity (transmitted-light intensity) of light received through the cuvette 80a and the sample in the cuvette 80a, and the signals are digitally converted by the A/D converter 22h and transmitted to the controller 24. The controller 24 receives the digital data continuously from a time point at which light application to the cuvette 80a is started, to a time when a predetermined measurement time (180 seconds) is reached. The digital data corresponds to the intensity of the received light, and is thus measurement values representing a degree of coagulation of the blood specimen, and the measurement values are obtained by receiving the digital data.

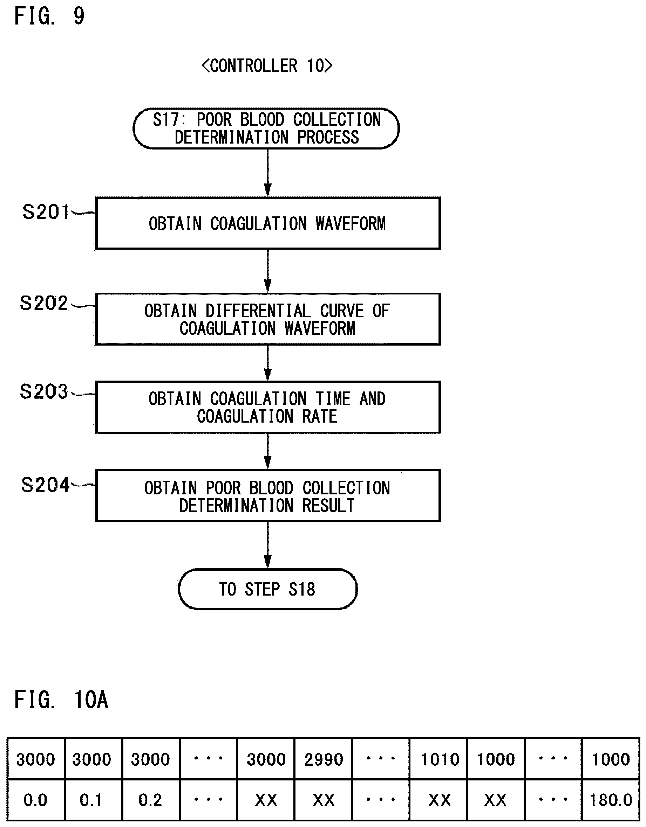

In step S104, the controller 24 obtains and stores the continuously received digital data as coagulation waveform data so as to associate the digital data with a time that has elapsed from the time point at which light application to the cuvette 80a has been started. A time interval at which the controller 24 receives the digital data is, for example, 0.1 seconds to 0.5 seconds. As shown in FIG. 10A, the coagulation waveform data is a two-dimensional array data set in which transmitted-light intensities are indicated as an example of the measurement values in the upper line, and times (time that has elapsed from a time point at which light application to the cuvette 80 has been started) at which each transmitted-light intensity is obtained is indicated are the lower line.

### 1.5 Poor blood collection determination process based on poor blood collection determination program (step S17)

When step S17 shown in FIG. 7 is started, the controller 10 performs a process of determining whether or not blood collection is poor as shown in FIG. 9. The process of performing determination as to poor blood collection is a process of performing determination as to poor blood collection of the blood specimen, based on the coagulation waveform data. The determination result as to poor blood collection may not necessarily be determination based on 100% accuracy. The determination indicating that blood collection is poor includes determination indicating that there is a possibility of poor blood collection.

In the process of determining whether or not blood collection is poor, values of a plurality of kinds of parameters concerning coagulation of a blood specimen are obtained on the basis of coagulation waveform data based on the measurement values representing a degree of coagulation, and determination as to poor blood collection of the blood specimen is performed based on the obtained values of the plurality of kinds of parameters. According to the invention, the plurality of kinds of parameters include one or more kinds of parameters concerning a coagulation rate of a blood specimen and one or more kinds of parameters concerning a coagulation time of a blood specimen. The number of the kinds of the parameters may be three or more.

The parameter concerning the coagulation time represents, for example, a coagulation time, a coagulation reaction start time, or a coagulation reaction end time. The coagulation time represents a time from a time when the calcium solution is added to a sample in which a blood specimen and the APTT measurement reagent are mixed, to a time when a predetermined coagulation state is reached. The coagulation reaction start time represents a time from addition of the calcium solution to a sample in which a blood specimen and the APTT measurement reagent are mixed, to the start of the coagulation reaction. The coagulation reaction end time represents a time from addition of the calcium solution to a sample in which a blood specimen and the APTT measurement reagent are mixed, to the end of the coagulation reaction. The parameter concerning the coagulation time may represent, for example, a time up to a time when the blood specimen reaches a predetermined state, such as a time from the coagulation reaction start time to the coagulation time. The parameter concerning the coagulation rate represents, for example, a maximum coagulation rate, a maximum coagulation acceleration, or a maximum coagulation deceleration. The maximum coagulation rate represents a maximum rate of the coagulation reaction. The maximum coagulation acceleration represents a maximum acceleration of the coagulation reaction rate. The maximum coagulation deceleration represents a maximum deceleration of the coagulation reaction rate.

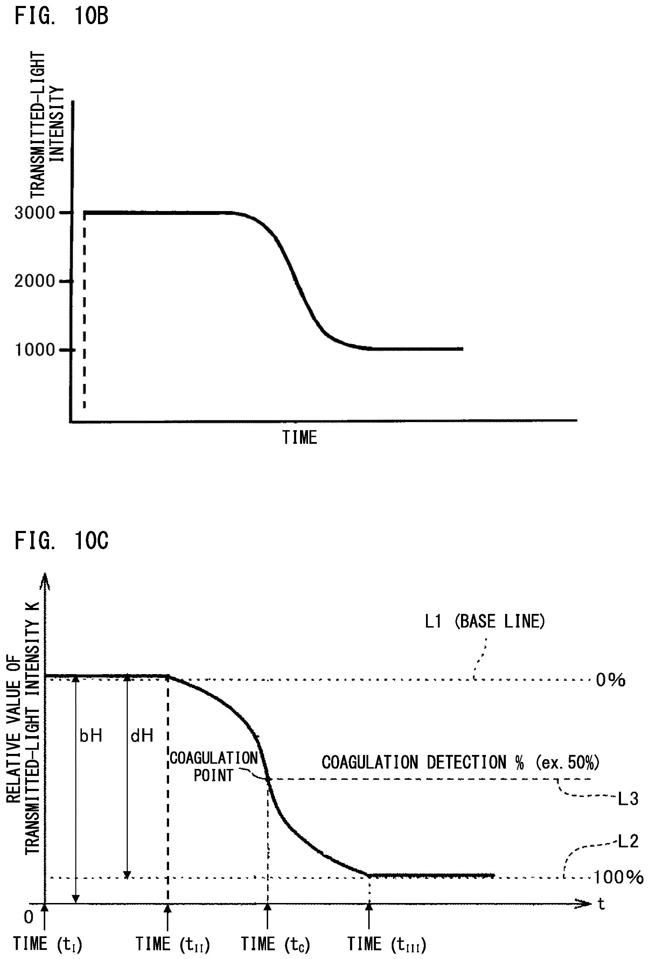

In step S201, the controller 10 operates to read out the coagulation waveform data stored in the storage unit 13 in step S16. The controller 10 operates to plot the coagulation waveform data having been read out, on a two-dimensional graph in which the vertical axis (Y axis) represents transmitted-light intensities, and the horizontal axis (X axis) represents measurement times (second: sec) for which the transmitted-light intensities are monitored, and generate a coagulation waveform as shown in FIG. 10B. Furthermore, the controller 10 operates to normalize the coagulation waveform. As shown in FIG. 10C, the normalized coagulation waveform is expressed such that each transmitted-light intensity included in the coagulation waveform data is indicated as a relative value so as to express the vertical axis between 0% (L1: base line) to 100% (L2).

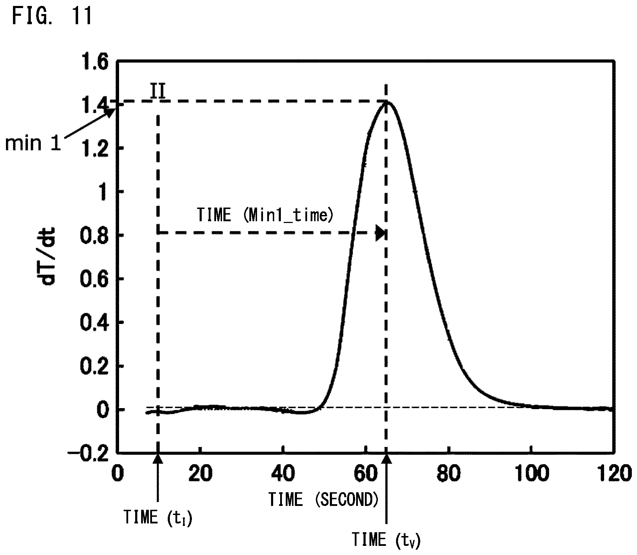

In step S202, the controller 10 operates to differentiate the normalized coagulation waveform to generate a first-order differential curve (differential coagulation waveform) of the coagulation waveform as shown in FIG. 11. In step S201, the normalization of the coagulation waveform may be omitted. In this case, the controller 10 operates to differentiate a coagulation waveform which is not normalized (see FIG. 10B), and thus obtain a first-order differential curve. In FIG. 11, the first-order differential curve is expressed such that the coagulation rate (dT/dt) represents a positive value. However, the first-order differential curve may be expressed such that the coagulation rate represents a negative value. That is, a curve in which the positive signs and the negative signs of the curve shown in FIG. 11 in the vertical axis are inverted to the opposite signs may be generated. The first-order differential curve is generated by differentiating a function representing the coagulation waveform with respect to time. The first-order differential curve may be based on a gradient (rate) calculated by dividing, on the basis of the coagulation waveform data that includes the measurement value obtained at a predetermined time interval, a difference for each measurement interval by the time interval thereof.

In step S203, the controller 10 obtains the coagulation time and the coagulation rate of the sample. The coagulation time can be obtained from the coagulation waveform. Obtaining the coagulation time from the coagulation waveform includes determining the coagulation time based on numerical data representing the coagulation waveform. The coagulation rate is obtained from the first-order differential curve of the coagulation waveform. Obtaining the coagulation rate from the first-order differential curve of the coagulation waveform includes determining the coagulation rate based on the numerical data representing the first-order differential curve. For example, calculated discrete rate data represents the first-order differential curve, and obtaining the coagulation rate from the first-order differential curve of the coagulation waveform includes obtaining the coagulation rate by determining the maximum rate from the discrete rate data.

Obtaining a coagulation time t_{c} based on the coagulation waveform data will be described with reference to FIG. 10C. The horizontal axis represents a time that elapses from a time when the measurement unit 200 starts applying light to a measurement sample in which a blood specimen and the blood coagulation measurement reagent are mixed. In the coagulation waveform data having been read out in step S201, a time t_{I} represents a time point at which light application to the cuvette 80a is started, and this time is set as 0. A difference between a time at which the calcium solution is added to the measurement sample, and a time at which light application is started is close to 0. Therefore, the time (0) can be regarded as a time point at which the calcium solution is added to the measurement sample. At the time t_{I}, fibrin is not deposited. Therefore, at the time t_{I}, the transmitted-light intensity indicates a high value. Thereafter, when the coagulation reaction progresses and deposition of fibrin starts, the deposited fibrin blocks light, and reduction of the transmitted-light intensity starts. This time point is a time t_{I I}, and is a coagulation reaction start time. The controller 10 obtains, through calculation, a time at which reduction of the transmitted-light intensity starts in the coagulation waveform, and sets the time as the time t_{I I}. The controller 10 sets an amount of change of the transmitted-light intensity at the time t_{I I} as 0%.

Thereafter, the transmitted-light intensity is further reduced according to progress of deposition of fibrin. When most of the fibrinogen in the sample has been converted to fibrin, the reaction converges, and the coagulation waveform plateaus. In step S203, the controller 10 obtains a time at which the coagulation waveform starts to plateau through calculation, and sets the time as a time t_{I I I}. The time t_{I I I} represents a coagulation reaction end time. The controller 10 sets an amount of change of the transmitted-light intensity at the time t_{I I I} as 100%. The controller 10 sets a time (time at an intersection of a line L3 and the coagulation waveform) at which an amount of change of the transmitted-light intensity is 50%, as the time t_{c} , and obtains the time t_{c} as the coagulation time. The coagulation time is not limited to a time when an amount of change of the transmitted-light intensity is 50%, and a time at which an amount of change of the transmitted-light intensity is any of, for example, 30%, 40%, 60%, 70%, or 100% may be obtained as the coagulation time. Alternatively, the coagulation time (time t_{c} ) may be, for example, a certain time counted from the time t_{I I} that is the coagulation reaction start time. In a case where normalization of the coagulation waveform is omitted, the controller 10 obtains the coagulation time t_{c} from a coagulation waveform which is not normalized.

The controller 10 can also obtain the value of the parameter concerning the coagulation time based on the first-order differential curve of the coagulation waveform which is generated in step S202. The first-order differential curve and the value of the parameter concerning the coagulation time will be described with reference to FIG. 11. A time (Min1_time) at which the maximum rate is reached is a time from a light application start time (time t_{I}) to a time (time t_{V} ) at which the coagulation rate becomes maximum, in the first-order differential curve.

In step S203, the controller 10 obtains the coagulation rate based on the first-order differential curve of the coagulation waveform which is generated in step S202. The coagulation rate is a coagulation rate at a time when the coagulation rate of the blood specimen reaches a predetermine rate. As shown in FIG. 11, |min 1| represents an absolute value of a peak value (min1) of the coagulation rate in the first-order differential curve, and represents the maximum coagulation rate. In the present embodiment, the maximum coagulation rate is obtained as the coagulation rate. In the present embodiment, graphs of the coagulation waveform and the first-order differential curve of the coagulation waveform are generated in order to obtain values of parameters concerning coagulation of the blood specimen. However, generation of a graph of the coagulation waveform and/or a graph of the first-order differential curve of the coagulation waveform may be omitted, and a value of the parameter concerning coagulation of the blood specimen may be obtained through calculation with respect to the coagulation waveform data. That is, in the present disclosure, step S201 and/or step S202 may be omitted.

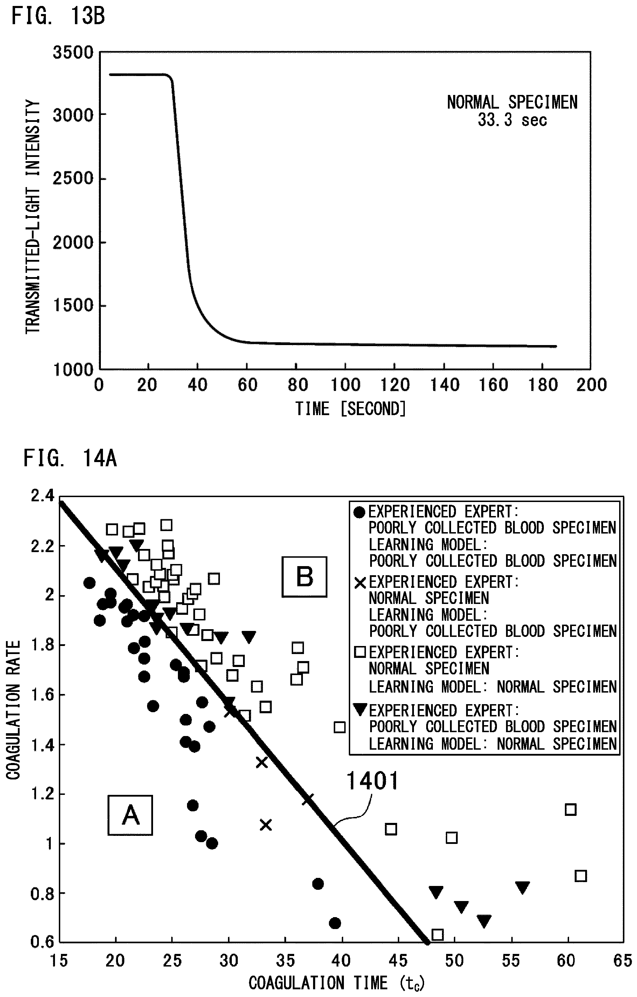

In step S204, the controller 10 determines whether or not blood collection is poor by determining whether or not blood collection is poor based on the coagulation time and the coagulation rate which have been obtained in step S203. In the present embodiment, whether or not blood collection is poor is determined by comparing the coagulation time and the coagulation rate of the blood specimen for which the determination is to be performed, with the criterion data 132 stored previously in the storage unit 13. In the present embodiment, as shown in FIG. 14A, the criterion data 132 includes data corresponding to a demarcation line 1401 in a scattergram in which the coagulation time and the coagulation rate are parameters. The controller 10 determines whether the blood specimen for which the determination is to be performed is positioned in a region A in which the coagulation time is shorter and the coagulation rate is lower than those in a region B, or in the region B in which the coagulation time is longer and the coagulation rate is higher than those in the region A, based on the coagulation time and the coagulation rate, and determines the blood specimen positioned in the region A as a specimen (hereinafter, referred to as "poorly collected blood specimen") in which blood collection is poor, and determines the blood specimen positioned in the region B as a specimen (hereinafter, referred to as "normal specimen") in which blood collection is not poor. If a tissue fluid containing a tissue factor is mixed in a syringe depending on a blood collection technique, blood coagulation is initiated and deposition of fibrin is started. Therefore, in the poorly collected blood specimen, since a coagulation factor is activated before the calcium solution is added, a time required from a time point at which the calcium solution is added, to the start of the coagulation reaction and the end of the coagulation reaction is considered to be shorter as compared with a normal specimen. Meanwhile, if the coagulation reaction is partially started before the calcium solution is added, since a component contributing to the blood coagulation has been consumed, a time required from the start of the coagulation reaction after addition of the calcium solution to the end of the coagulation reaction becomes longer as compared with a normal specimen, that is, the coagulation rate is considered to be low. Thus, in a case where a tissue fluid is mixed in the blood specimen due to poor blood collection, a value of the parameter concerning the coagulation time and a value of the parameter concerning the coagulation rate are considered to be affected. Therefore, it is considered that whether or not blood collection is poor can be determined based on the parameter concerning the coagulation time and the parameter concerning the coagulation rate. A method for determining data corresponding to the demarcation line 1401 will be described below.

Subsequently, in step S18 (FIG. 7), the controller 10 obtains an analytic value concerning coagulability of a blood specimen to be tested, in order to use the analytic value for, for example, grasping a pathological condition for which the coagulation test is performed. In the present embodiment, the coagulation time (APTT) is obtained as an analytic value for a test in which a hemostatic function is predicted. The coagulation time (APTT) is a time from addition of calcium to a time when the blood specimen reaches a predetermined coagulation state. The analytic value may be a time from start of coagulation reaction of the blood specimen to a time when the blood specimen reaches a predetermined coagulation state.

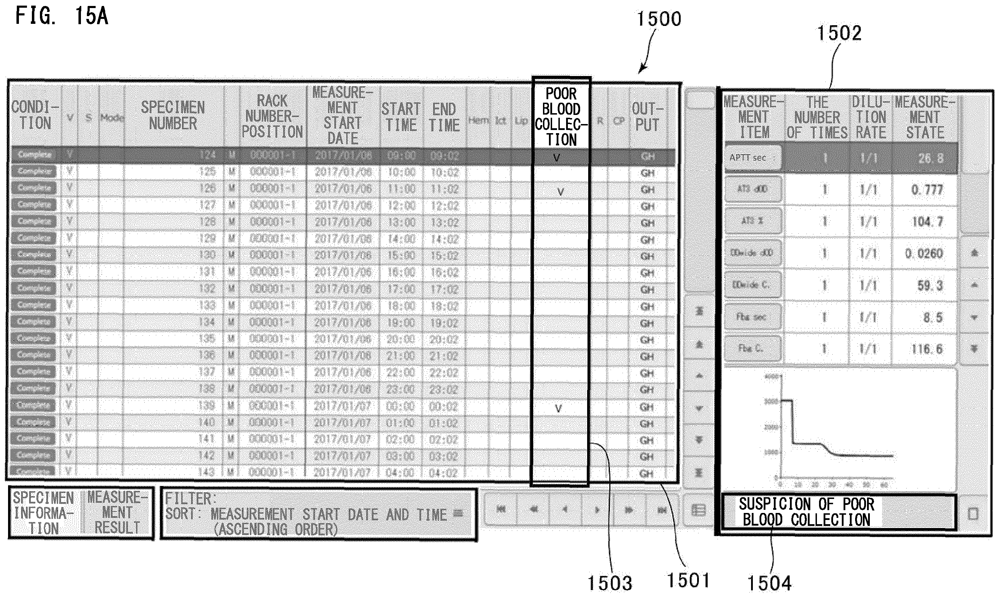

When the controller 10 obtains the analytic value in step S18 (FIG. 7), the controller 10 causes the output unit 17 to output the determination result as to poor blood collection as obtained in step S17 and the analysis result including the analytic value obtained in step S18, in step S19 (FIG. 7). FIGS. 15A to 15D show examples of output screens on which the output unit 17 displays the analysis results. FIG. 15A shows an analysis result list display screen 1500 that includes a list display region 1501 for displaying a list of a plurality of analyzed blood specimens, and an individual overview display region 1502 for displaying overview information of an analysis result of the blood specimen selected based on input from the user in the list display region 1501. The list display region 1501 includes a plurality of lines, and information of the blood specimens is indicated in the respective lines.

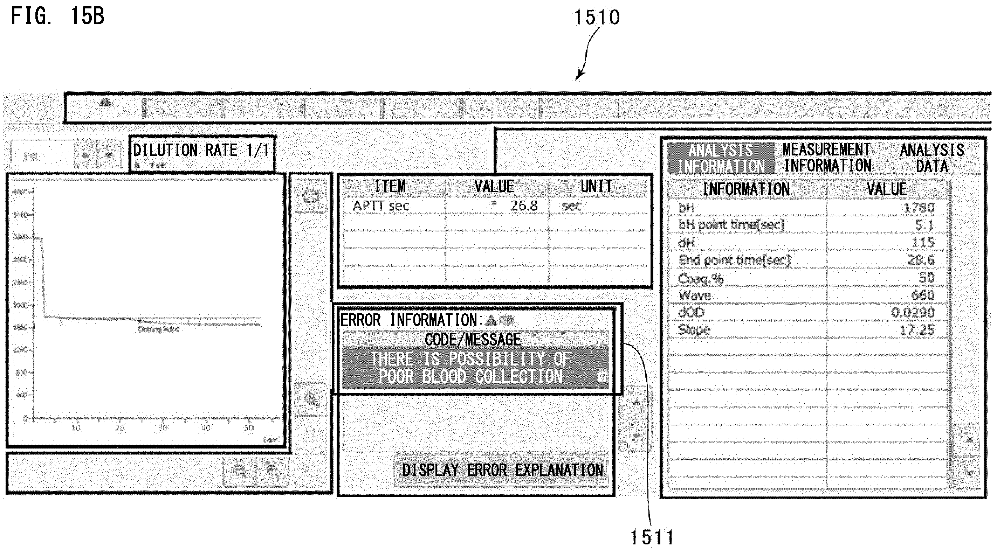

A plurality of columns in the list display region 1501 include, as one column, a poor blood collection indication column 1503 indicating a blood specimen for which blood collection is determined to be poor. In the example shown in FIG. 15A, a blood specimen in a line in which "V" is displayed in the poor blood collection indication column 1503 is suspected of poor blood collection. This is displayed based on the determination result as to poor blood collection as obtained in the poor blood collection determination process of step S17. That is, information indicating that there is suspicion of poor blood collection is indicated for the blood specimen for which blood collection is determined to be poor in the poor blood collection determination process. In a poor blood collection indication region 1504 of the individual overview display region 1502, information indicating that there is suspicion of poor blood collection is displayed. FIG. 15B shows an example of a screen 1510 for displaying information of the analysis result of one blood specimen in detail. The screen 1510 includes a poor blood collection indication region 1511, and information indicating that the blood specimen having its analysis result displayed therein is suspected of poor blood collection, is displayed.

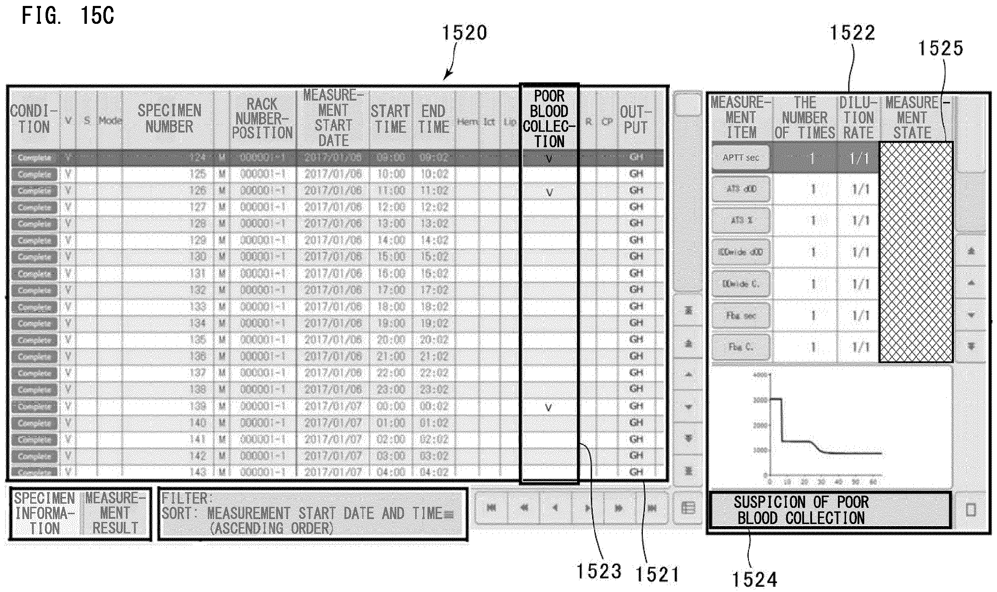

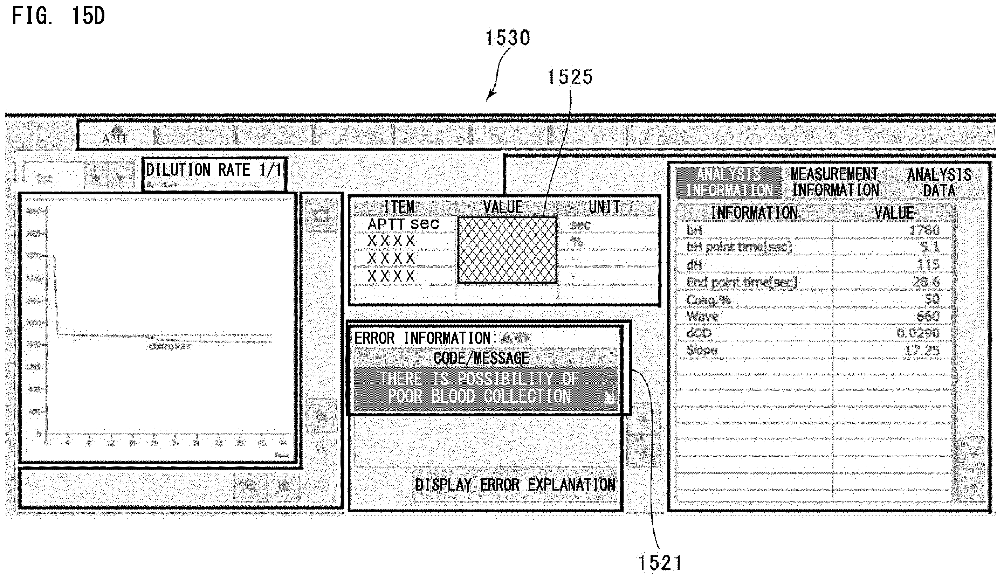

FIG. 15C and FIG. 15D show modifications of output screens for displaying an analysis result. FIG. 15C shows a modification of the screen in FIG. 15A, and a mask 1525 instead of analytic values (each of analytic values such as APTT and AT3) of the blood specimen for which blood collection is determined to be poor is displayed in an individual overview display region 1522 without displaying the analytic values. That is, output of an analytic value of the blood specimen for which blood collection is determined to be poor is prohibited. For example, when the process of displaying the analysis result is performed, whether or not the result of the poor blood collection determination indicates that blood collection is poor, is determined, and, when the blood collection is determined to be poor, the masked image is outputted instead of a value indicating the analytic value being displayed. Similarly, also on a screen 1530 for displaying information of the analysis result of one blood specimen in detail as shown in FIG. 15D, the mask 1525 is displayed instead of the APTT analytic value, and output of the analytic value is thus prohibited. By prohibiting display of the analytic value used for, for example, grasping a pathological condition by using a coagulation test, a pathological condition or the like is prevented from being erroneously grasped as an analytic value obtained from a normal specimen although the specimen is suspected of poor blood collection. In FIG. 15D, analytic values of other measurement items are also masked (indicated as xxx in FIG. 15D) in addition to the APTT value. In a case where there is suspicion of poor blood collection, output of analytic values of measurement items other than the measurement item used for determination as to poor blood collection is also prohibited, whereby a pathological condition or the like is more assuredly prevented from being erroneously grasped as an analytic value obtained from a normal specimen.

A method for determining the demarcation line 1401 (FIG. 14A) as an example of the criterion data 132 to be stored in the storage unit 13 will be described below. The demarcation line 1401 is determined when a developer performs the following procedure in the development stage of the analysis system 1000.

In the present embodiment, one poorly collected blood specimen and one normal specimen are collected and prepared from each of a plurality of donors. The coagulation time and the coagulation rate are obtained for each of the plurality of poorly collected blood specimens and the plurality of normal specimens, a scattergram of the coagulation time and the coagulation rate is generated, and a demarcation line as a boundary between the poorly collected blood specimens and the normal specimens is determined based on the generated scattergram. For the poorly collected blood specimens, an experienced expert of the test selects specimens for which blood collection is confirmed to be poor, based on criteria for determining that blood collection is poor, in a site of a clinical laboratory test, such as presence or absence of clots formed in the blood specimen and presence or absence of divergence between the analytic value and clinical information. For the normal specimens, specimens are collected again from the donors from whom the specimens confirmed as the poorly collected blood specimens have been collected, and the experienced expert of the test selects specimens for which blood collection is not confirmed to be poor, based on the criteria for determining that the blood collection is poor.

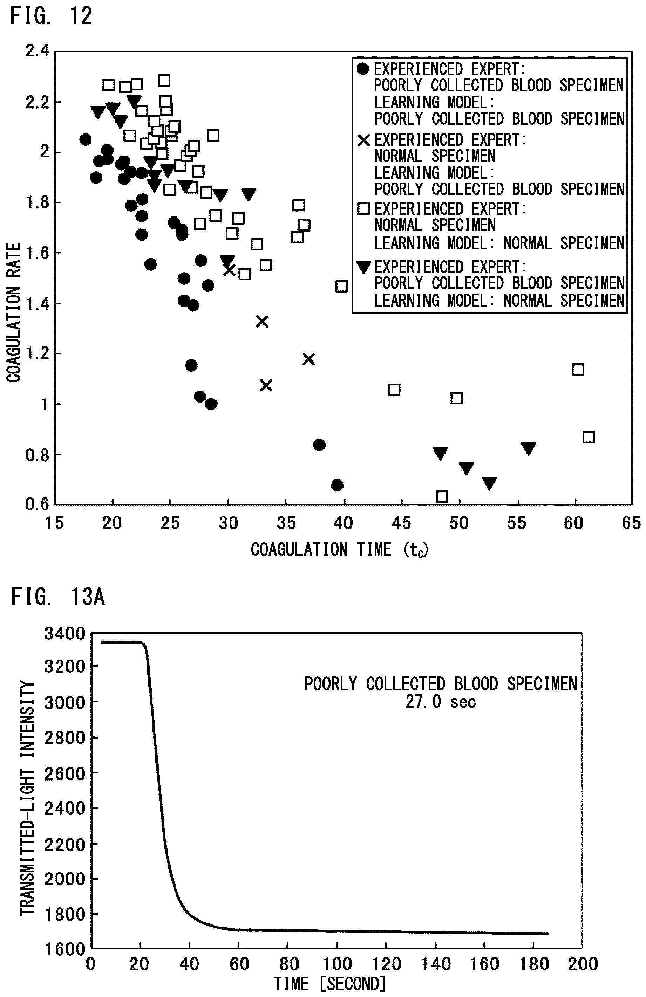

The coagulation time and the coagulation rate of each specimen for generating a scattergram are obtained in the same method as is performed in steps S11 to S16 and steps S201 to S203 described above. FIG. 12 shows an example of the generated scattergram. In FIG. 12, • and ▼ represent specimens selected as poorly collected blood specimens by the experienced expert, and □ and × represent specimens selected as normal specimens by the experienced expert. • represents a specimen determined as a poorly collected blood specimen also by a learning model generated by the method of the present embodiment, and ▼ represents a specimen determined as a normal specimen by the learning model of the present embodiment. □ represents a specimen determined as a normal specimen also by the learning model of the present embodiment. × represents a specimen determined as a poorly collected blood specimen by the learning model of the present embodiment.

FIG. 13A shows an example of a coagulation waveform of a poorly collected blood specimen, and FIG. 13B shows an example of a coagulation waveform of a normal specimen collected from the donor who is the same as the donor of the poorly collected blood specimen in FIG. 13A. The coagulation time of the poorly collected blood specimen is 27.0 seconds whereas the coagulation time of the normal specimen is 33.3 seconds. The gradient of the coagulation waveform from the start of the coagulation reaction is gentler in FIG. 13A. Therefore, it is found that the coagulation rate is lower in the poorly collected blood specimen. It is considered that, since a tissue fluid is mixed in the specimen, the coagulation time is shortened and the coagulation rate is lowered in the poorly collected blood specimen shown in FIG. 13A. This tendency is found in the scattergram shown in FIG. 12. That is, the coagulation time is shorter and the coagulation rate is lower in the poorly collected blood specimen. Therefore, the poorly collected blood specimens are distributed in the lower-left side in the scattergram, and the normal specimens are distributed in the upper-right side in the scattergram. Based on this tendency, the demarcation line is determined to classify the specimens into the poorly collected blood specimens and the normal specimens.

Classification into the poorly collected blood specimens and the normal specimens can be performed by using the demarcation line. The demarcation line is determined by machine learning using a linear SVM (Support Vector Machine) in which the coagulation rates and the coagulation times obtained from the poorly collected blood specimens and the normal specimens are used as training data. A learned model including data corresponding to the determined demarcation line is stored as a part of the criterion data 132 in the storage unit 13. The linear SVM is one of pattern recognition models generated by machine learning with use of training data. As the linear SVM, a generally known one can be used. The demarcation line may be determined by using a non-linear SVM or another pattern recognition model, or determined by a system designer. As a technique for classifying the blood specimens to be tested into poorly collected blood specimens and normal specimens, a general classification method such as a decision tree, Bayesian classification, logistic regression, and neural network may be used.

FIG. 14A shows the determined demarcation line 1401 on the scattergram shown in FIG. 12. The demarcation line 1401 is determined by machine learning using the linear SVM in which the coagulation rates and the coagulation times obtained from the poorly collected blood specimens and the normal specimens as shown in FIG. 12 are used as the training data. By determining a region including the plotted position that is determined by the coagulation time and the coagulation rate obtained from a blood specimen to be tested, among regions obtained by dividing the scattergram by the demarcation line, whether the blood specimen is a poorly collected blood specimen or a normal specimen can be determined. The region below the demarcation line 1401 is set as the region A in which the specimen is determined as a poorly collected blood specimen, and the region above the demarcation line 1401 is set as the region B in which the specimen is determined as a normal specimen. In a case where the plotted position of the blood specimen to be tested is determined to be positioned in the region A, the blood specimen is determined as a poorly collected blood specimen. In a case where the plotted position of the blood specimen to be tested is determined to be positioned in the region B, the blood specimen is determined as a normal specimen.

### (Example 1)

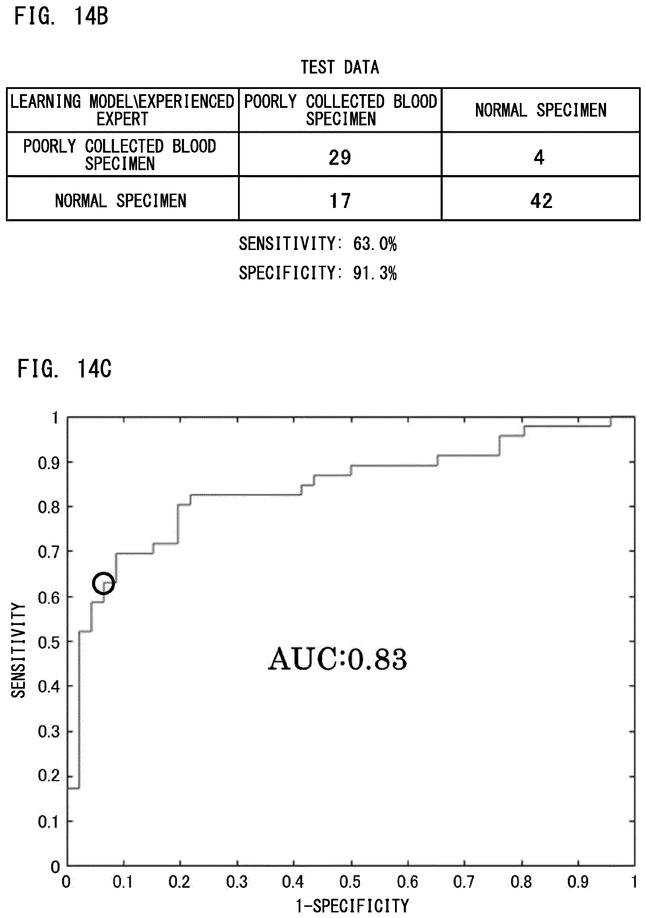

FIG. 14B shows an example in which the poor blood collection determination process was performed by using the learned model based on the demarcation line shown in FIG. 14A. An experienced expert of the test selected poorly collected blood specimens and normal specimens according to the criteria and a selection method for determining poor blood collection in the site of the clinical laboratory test as described above, to prepare 46 sets of paired poorly collected blood specimen and normal specimen (92 specimens). Revohem (Registered Trademark) APTT SLA (SYSMEX CORPORATION) was used as the APTT measurement reagent, to measure each specimen, and the coagulation rate and the coagulation time were obtained for each specimen. The coagulation rate and the coagulation time of each specimen were used as training data, to determine the demarcation line 1401 (FIG. 14A) by machine learning using the linear SVM. The poor blood collection determination process was performed by using the learned model including the demarcation line 1401, and whether the specimen was a poorly collected blood specimen or a normal specimen was determined based on the coagulation rate and the coagulation time.

In FIG. 14B, the poorly collected blood specimens in the second column represent poorly collected blood specimens selected by the experienced expert, and the number of the specimens was 46 (=29+17). The normal specimens in the third column represent normal specimens selected by the experienced expert, and the number of the specimens was 46 (=4+42). The poorly collected blood specimens in the second line represent poorly collected blood specimens determined by the poor blood collection determination process using the learned model, and the number of the specimens was 33 (=29+4). The normal specimens in the third line represent normal specimens determined by the poor blood collection determination process using the learned model, and the number of the specimens was 59 (=17+42). A ratio of the number of the specimens determined as poorly collected blood specimens by both the experienced expert and the learned model, relative to the number of the specimens determined as poorly collected blood specimens by the experienced expert represents a sensitivity, and the sensitivity was 63.0% (=29/46×100) in this example. A ratio of the number of the specimens determined as normal specimens by both the experienced expert and the learned model, relative to the number of the specimens determined as normal specimens by the experienced expert represents a specificity, and the specificity was 91.3% (=42/46×100) in this example. FIG. 14C shows an ROC curve generated based on the above-described pairs for evaluating fractionation performance of the learning model. The AUC was 0.83 and the fractionation performance was confirmed to be high. In FIGS. 12 and 14A, • represents a specimen determined as a poorly collected blood specimen by both the experienced expert and the learned model, × represents a specimen determined as a normal specimen by the experienced expert but determined as a poorly collected blood specimen by the learned model, □ represents a specimen determined as a normal specimen by both the experienced expert and the learned mode, and ▼ represents a specimen determined as a poorly collected blood specimen by the experienced expert but determined as a normal specimen by the learned model.

### (Modification 1)

In Modification 1 of the present embodiment, instead of obtaining the differential curve of the coagulation waveform and a parameter value concerning the coagulation waveform based on the differential curve in steps S202 and S203 in FIG. 9, fitting of a mathematical model to the coagulation waveform is performed, and values of parameters concerning the coagulation time and the coagulation rate are obtained based on the fitted mathematical model. The predetermined mathematical model used in this modification is a sigmoid function described below. The predetermined mathematical model is not limited to a sigmoid function as long as the shape of the coagulation waveform can be expressed. Examples of the mathematical model include a Gompertz function, a cumulative normal distribution function, and a Gudermannian function.$f \left(x\right) = α \left(1-\left(\frac{1}{1 + {e}^{- β \left(x-t\right)}}\right)\right) + δ$

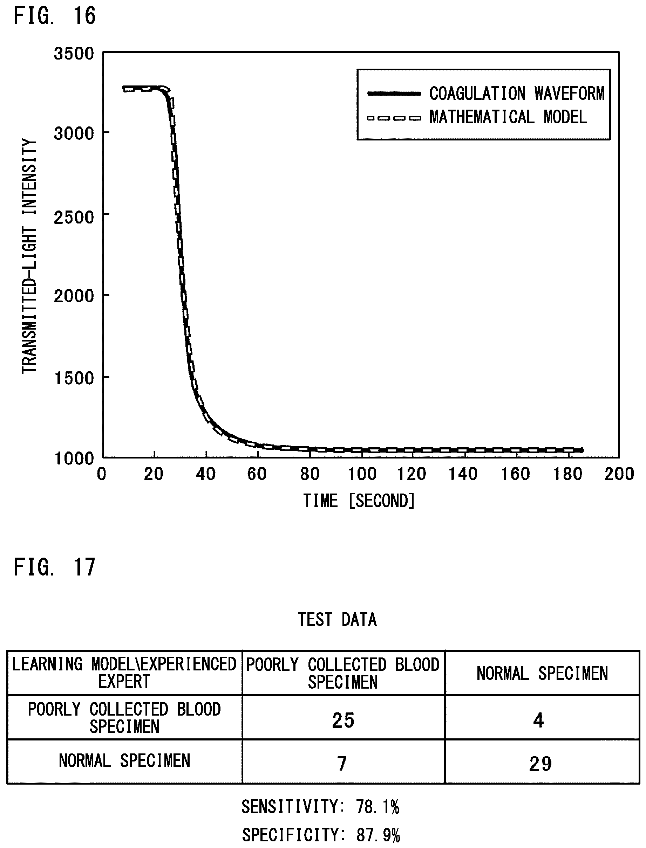

The controller 10 performs fitting of the sigmoid function represented by Math. 1 (equation 1) to the coagulation waveform data having been read out in step S201, and obtains a value of each parameter from values of coefficients obtained by the fitting. FIG. 16 shows an example of a waveform obtained when fitting of the equation 1 to the coagulation waveform is performed. α of the sigmoid function in the waveform obtained by the fitting process is an amplitude of a reaction waveform, β is a reaction speed at an inflection point, t is an inflection point of the reaction waveform, and δ is a minimum value of the transmitted-light intensity. Here, t is the parameter concerning the coagulation time, β is the parameter concerning the coagulation rate, and values of t and β are obtained. α (amplitude of reaction waveform) is considered to be almost equal to a difference (dH) between L 1 and L2 of the relative values of the transmitted-light intensities in FIG. 10C, and δ is considered to be almost equal to a height (bH-dH) of L2, and these values can thus be handled. The method of fitting of the mathematical model has high robustness against influence due to noise and abnormal waveforms such as an abnormal ERE waveform. Furthermore, the method is advantageous in that expandability allowing various waveform characteristics to be quantified by changing the mathematical model, can be provided. Step S204 and the subsequent steps are the same as those in the first embodiment.

### (Example 2)

FIG. 17 shows an example of Modification 1. The experienced expert of the test selected poorly collected blood specimens and normal specimens according to the criteria and a selection method for determining poor blood collection in the site of the clinical laboratory test as described above, to prepare 32 sets of paired poorly collected blood specimen and normal specimen (64 specimens). Revohem (Registered Trademark) APTT SLA (SYSMEX CORPORATION) was used as the APTT measurement reagent, to measure each specimen, and the coagulation rate and the coagulation time were obtained for each specimen. t and β of each specimen were used as training data, to determine the demarcation line by machine learning using the linear SVM. The poor blood collection determination process was performed by using the learned model including the demarcation line, and whether the specimen was a poorly collected blood specimen or a normal specimen was determined based on t and β.

In FIG. 17, the poorly collected blood specimens in the second column represent poorly collected blood specimens selected by the experienced expert, and the number of the specimens was 32 (=25+7). The normal specimens in the third column represent normal specimens selected by the experienced expert, and the number of the specimens was 33 (=4+29). The poorly collected blood specimens in the second line represent poorly collected blood specimens determined by the poor blood collection determination process using the learned model, and the number of the specimens was 29 (=25+4). The normal specimens in the third line represent normal specimens determined by the poor blood collection determination process using the learned model, and the number of the specimens was 36 (=7+29). A ratio of the number of the specimens determined as the poorly collected blood specimens by both the experienced expert and the learned model, relative to the number of the specimens determined as the poorly collected blood specimens by the experienced expert represents a sensitivity, and the sensitivity was 78.1% (=25/32×100) in this example. A ratio of the number of the specimens determined as the normal specimens by both the experienced expert and the learned model, relative to the number of the specimens determined as the normal specimens by the experienced expert represents a specificity, and the specificity was 87.9% (=29/33 × 100) in this example. The sensitivity and the specificity indicated high values, and fractionation performance was confirmed to be high also in this modification.

### (Modification 2)

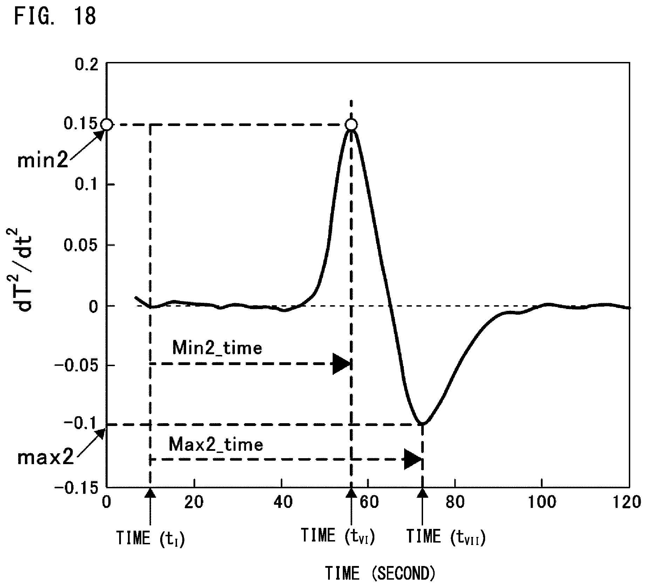

As Modification 2 of the present embodiment, an example in which another parameter concerning coagulation is used will be described. In this modification, a parameter can also be obtained based on a second-order differential curve obtained by further differentiating the first-order differential curve of the coagulation waveform. FIG. 18 shows an example of the second-order differential curve obtained based on the first-order differential curve in FIG. 11.

Another parameter concerning coagulation will be described with reference to FIG. 10C, FIG. 11, and FIG. 18. Examples of the parameter concerning the coagulation time include a maximum rate arrival time (Min1_time) (FIG. 11), a maximum acceleration arrival time (Min2_time) (FIG. 18), and a maximum deceleration time (Max2_time) (FIG. 18), in addition to the coagulation time. However, the parameter concerning the coagulation time is not limited thereto. As described above, the maximum rate arrival time (Min1_time) represents a time from a light application start time (time t_{I} ) to a time (time t_{V} ) at which the coagulation rate becomes maximum, in the first-order differential curve. The maximum acceleration arrival time (Min2_time) represents a time from the light application start time (time t_{I} ) to a time (t_{V I} ) at which the coagulation acceleration becomes maximum in the second-order differential curve. The maximum deceleration time (Max2 _time) represents a time from the light application start time (time tI) to a time (t_{V I I} ) at which the coagulation deceleration becomes maximum in the second-order differential curve. The maximum rate arrival time (Min1_time) is almost equal to the coagulation time (time t_{c}) obtained based on the coagulation waveform data, and the maximum rate arrival time (Min1_time) can thus be handled as the coagulation time (time t_{c} ). Similarly, the maximum acceleration arrival time (Min2_time) is almost equal to the coagulation reaction start time (time t_{I I} ), and the maximum deceleration time (Max2_time) is almost equal to the coagulation reaction end time (time t_{I I I} ), and the maximum acceleration arrival time (Min2_time) and the maximum deceleration time (Max2_time) can thus be handled as the coagulation reaction start time (time t_{I I} ) and the coagulation reaction end time (time t_{I I I} ), respectively.

Examples of the values of the parameters concerning the coagulation rate include |min 1|, |min 2|, and |max 2|. However, the value of the parameter is not limited thereto. As described above, |min 1| represents an absolute value of a peak value (min1) (FIG. 11) of the coagulation rate in the first-order differential curve, and represents the maximum coagulation rate. As shown in FIG. 18, |min 2| represents an absolute value of a peak value (min2) of the coagulation acceleration in the second-order differential curve, and represents the maximum coagulation acceleration. |max 2| represents an absolute value of a peak value (max2) of the coagulation deceleration in the second-order differential curve, and represents the maximum coagulation deceleration.

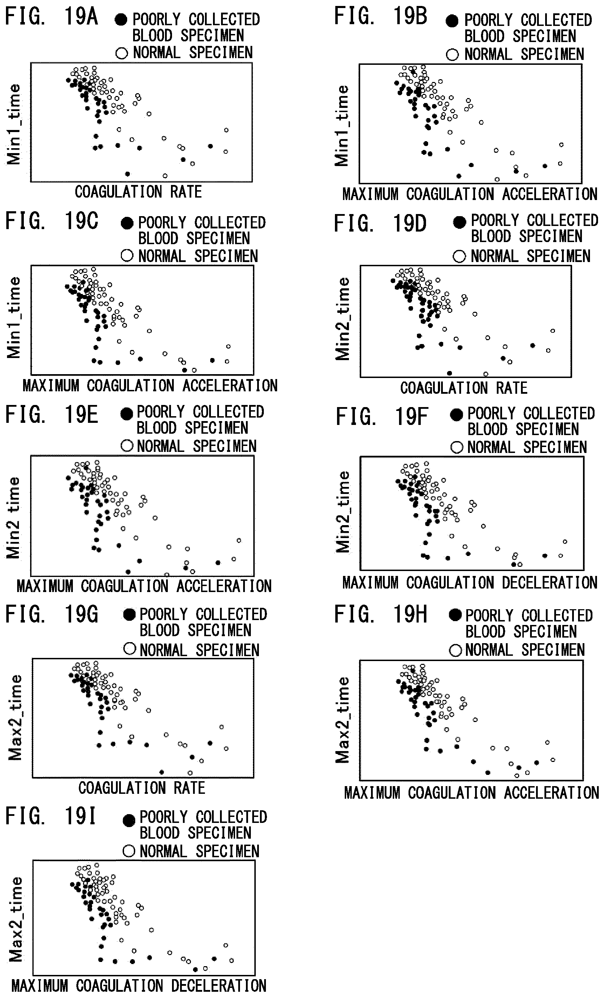

FIGS. 19A to 19I each illustrate a scattergram of poorly collected blood specimens and normal specimens in a case where the vertical axis and the horizontal axis represent the parameters concerning the coagulation time and the coagulation rate. In each scattergram, the vertical axis represents the parameter concerning the coagulation time and the horizontal axis represents the parameter concerning the coagulation rate. In the scattergram, • represents a poorly collected blood specimen and ∘ represents a normal specimen. According to the scattergram illustrated in each of FIGS. 19A to 19I, it is found that the poorly collected blood specimens and the normal specimens tend to be each distributed in a certain region. In these examples, the poorly collected blood specimens tend to be distributed in a lower-left region in the scattergram, and the normal specimens tend to be distributed in the upper-right region. Therefore, it can be understood that, also in the scattergram in which these parameters concerning the coagulation time and the coagulation rate are used, the poor blood collection determination can be performed for a blood specimen to be tested by the same technique as in the above-described embodiment and modification.

### (Modification 3)

In Modification 3 of the present embodiment, the poor blood collection determination process is performed by using three parameters concerning coagulation. Preferably, one of the three parameters concerning the coagulation waveform is the parameter concerning the coagulation time, and another parameter is the parameter concerning the coagulation rate. The other parameter may be a parameter concerning the coagulation time, a parameter concerning the coagulation rate, or a parameter concerning neither of them.

In this modification, similarly to Modification 1, fitting of the sigmoid function to the coagulation waveform is performed, and α, β, and t in the fitted sigmoid function are set as three parameters concerning the coagulation time and the coagulation rate. α represents an amplitude of the reaction waveform, β represents a reaction speed at an inflection point, and t represents an inflection point of the reaction waveform. In this modification, as one of the three parameters concerning the coagulation waveform, t is adopted as the parameter concerning the coagulation time, and β is adopted as the parameter concerning the coagulation rate.

### (Example 3)

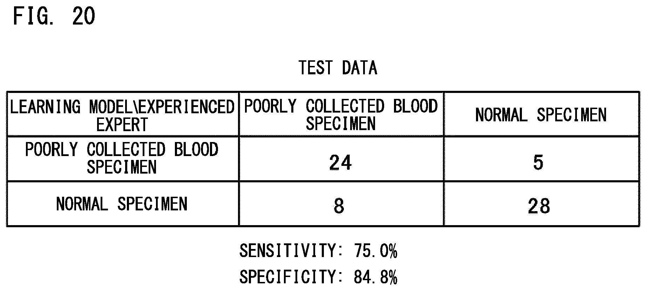

FIG. 20 shows an example of Modification 3. The experienced expert of the test selected poorly collected blood specimens and normal specimens according to the criteria and a selection method for determining poor blood collection in the site of the clinical laboratory test as described above, to prepare 32 sets of paired poorly collected blood specimen and normal specimen (64 specimens). Revohem (Registered Trademark) APTT SLA (SYSMEX CORPORATION) was used as the APTT measurement reagent, to measure each specimen, and the coagulation rate and the coagulation time were obtained for each specimen. t, α, and β of each specimen were used as training data, to determine the demarcation line by machine learning using the linear SVM. The poor blood collection determination process was performed by using the learned model including the demarcation line, and whether the specimen was a poorly collected blood specimen or a normal specimen was determined based on t, α, and β.

In FIG. 20, the poorly collected blood specimens in the second column represent poorly collected blood specimens selected by the experienced expert, and the number of the specimens was 32 (=24+8). The normal specimens in the third column represent normal specimens selected by the experienced expert, and the number of the specimens was 33 (=5+28). The poorly collected blood specimens in the second line represent poorly collected blood specimens determined by the poor blood collection determination process using the learned model, and the number of the specimens was 29 (=24+5). The normal specimens in the third line represent normal specimens determined by the poor blood collection determination process using the learned model, and the number of the specimens was 36 (=8+28). A ratio of the number of the specimens determined as poorly collected blood specimens by both the experienced expert and the learned model, relative to the number of the specimens determined as poorly collected blood specimens by the experienced expert represents a sensitivity, and the sensitivity was 75.0% (=24/32×100) in this example. A ratio of the number of specimens determined as normal specimens by both the experienced expert and the learned model, relative to the number of specimens determined as normal specimens by the experienced expert represents a specificity, and the specificity was 84.8% (=28/33×100) in this example. The sensitivity and the specificity indicated high values, and fractionation performance was confirmed to be high also in this modification.

### (Modification 4)

In Modification 4 of the present embodiment, the controller 10 performs steps S11, S12, S16, and S17 but does not perform a process step of step S18 for obtaining an analytic value. Furthermore, the controller 10 merely performs a process of outputting and storing the determination result as to poor blood collection, in step S19. An analysis system for merely performing the poor blood collection determination process can be provided.

### (Modification 5)

In Modification 5 of the present embodiment, the controller 10 instead of the controller 24 operates to obtain the coagulation waveform data based on the measurement value. The controller 24 does not operate to perform step S104 after step S103, and operates to transmit each measurement value obtained in step S103 to the analyzer 100 in step S15. The controller 10 operates to receive each measurement value from the measurement unit 200, associate each of the received measurement values with a time that has elapsed from the light application, and store the obtained measurement values as the coagulation waveform data in the storage unit 13, in step S16. Thereafter, the controller 10 performs step S17 and the subsequent process steps.

### (Second embodiment)

The present embodiment is different from the first embodiment in that a server 102 instead of the analyzer 100 performs the poor blood collection determination process (step S17) in the present embodiment. The system of the present embodiment includes, instead of the analyzer 100, a client device 101 which does not perform the poor blood collection determination process. Difference from the first embodiment will be mainly described below. The components which are not described in the present embodiment are the same as those of the first embodiment. The client device 101 may perform or may not perform the analytic value obtaining process (process step of step S18). In a case where the client device 101 does not perform the analytic value obtaining process, the server 102 may perform the analytic value obtaining process. In an analysis system 2000 described below, the client device 101 does not perform the analytic value obtaining process and the server 102 performs the analytic value obtaining process.

### 2. Analysis system 2000

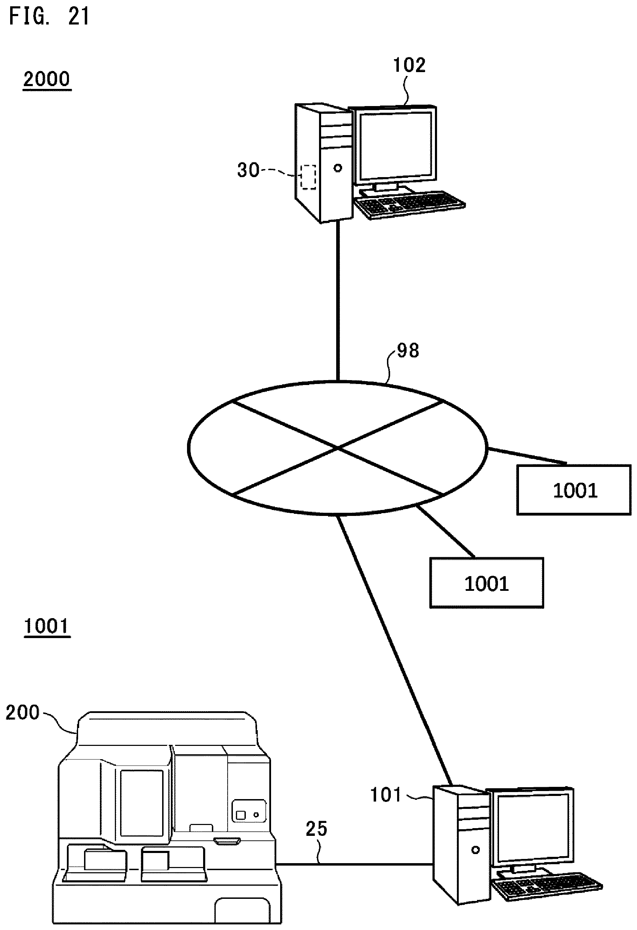

FIG. 21 shows an outer appearance of the analysis system 2000 of the present embodiment. The analysis system 2000 includes a plurality of analysis subsystems 1001 for obtaining and outputting measurement data of the blood specimens, and the server 102 for performing coagulation analysis for the coagulation waveform data of the blood specimens which are obtained by the plurality of analysis subsystems 1001. The analysis subsystem 1001 includes the client device 101 for controlling the measurement unit 200 and outputting a coagulation analysis result of the blood specimen, and the measurement unit 200 for applying light to a blood specimen that has started the coagulation reaction, obtaining optical information, and obtaining coagulation waveform data. The client device 101 and the measurement unit 200 are connected to each other via the communication cable 25. The server 102 is connected to a plurality of the client devices 101 via the network 98. The network is, for example, a local area network (LAN). The network 98 may be the Internet.

The client device 101 is the same as the analyzer 100 only except that the client device 101 does not perform the poor blood collection determination process and the analytic value obtaining process. The configurations of the server 102 and the client device 101 are the same as the configuration of the analyzer 100 shown in FIG. 2. The server 102 includes a controller 30. The controller 30 includes a CPU, a DRAM, a SRAM, a solid-state drive, and Ethernet, similarly to the controller 10. In the storage unit of the controller 30 of the server 102, the poor blood collection determination program, the analysis program, and the criterion data are stored. In the storage unit of the client device 101, a program for outputting an analysis result including a poor blood collection determination result is stored.

### 2.1 Measurement process, poor blood collection determination process, analytic value obtaining process, and output process

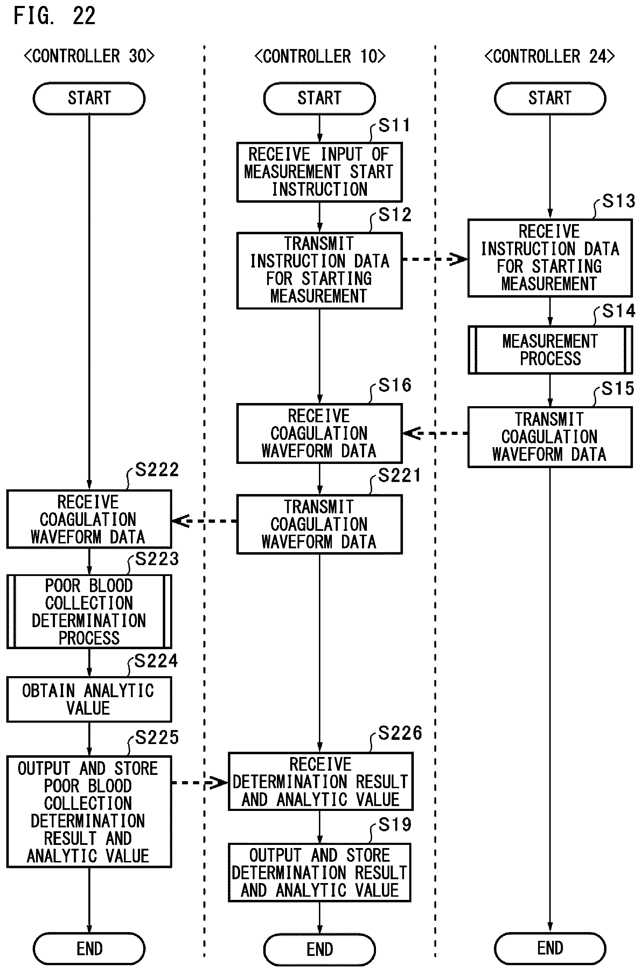

Flows of the measurement process, the poor blood collection determination process, the analytic value obtaining process, and the output process according to the present embodiment will be described with reference to FIG. 22. The same process steps as shown in FIG. 7 are denoted by the same reference characters as in FIG. 7. Similarly to the first embodiment, when the controller 10 receives a measurement start instruction inputted through the input unit 16 by the user in step S11, the controller 24 operates to perform the measurement process, and the controller 10 operates to receive the coagulation waveform data (S12 to S16). Thereafter, the controller 10 does not perform the poor blood collection determination process and the analytic value obtaining process, and the controller 10 operates to transmit the received coagulation waveform data to the server 102 in step S221. When the coagulation waveform data is received in step S222, the controller 30 performs the poor blood collection determination process in step S223. The poor blood collection determination process to be performed in the present embodiment is the same as the poor blood collection determination process shown in FIG. 9 except that the server 102 performs the poor blood collection determination process in the present embodiment. The controller 30 performs the analytic value obtaining process in step S224. The analytic value obtaining process to be performed in the present embodiment is the same as the analytic value obtaining process shown in FIG. 7 except that the server 102 performs the analytic value obtaining process in the present embodiment. In step S225, the server 102 operates to transmit the obtained poor blood collection determination result and analytic value, to the client device 101. The controller 10 operates to receive the poor blood collection determination result and the analytic value in step S226, and performs a process of outputting and storing the poor blood collection determination result and the analytic value in step S19.

### 2.2 Process according to Modification

In Modification of the second embodiment, the controller 10 may operate to perform the process steps up to the process of obtaining the coagulation time and the coagulation rate (up to step S203), and the controller 10 may operate to transmit, to the server 102, values of a plurality of parameters concerning coagulation of the blood specimen, for example, values of the coagulation time and the coagulation rate, instead of transmitting the coagulation waveform data. In this case, when the server 102 receives the values of the coagulation time and the coagulation rate, the server 102 operates to perform the poor blood collection determination (S204), the analytic value obtaining process (S224), and the subsequent process steps.

### 2.3 Parameter according to Modification

In Modification of the second embodiment, the controller 30 performs the poor blood collection determination process by using the parameters described in Modifications 1 to 3 of the first embodiment.

### (Third embodiment)

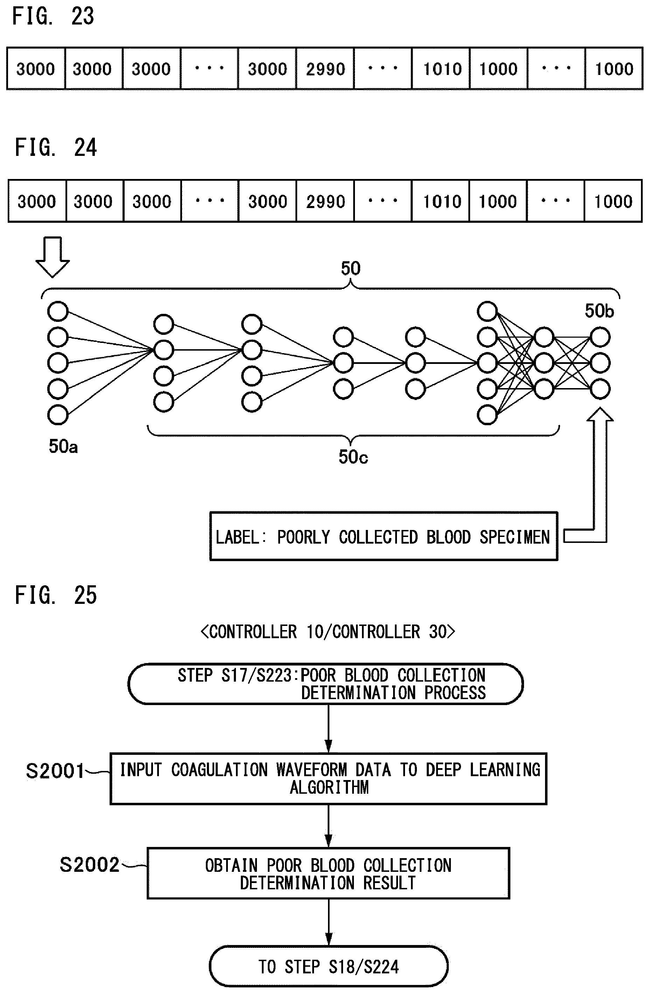

The present embodiment is different from the first embodiment and the second embodiment in that, in step S104, one-dimensional array data in which transmitted-light intensities as one example of the measurement value are chronologically arrayed is obtained as the coagulation waveform data, and the coagulation waveform data is inputted to the learned deep learning algorithm in the poor blood collection determination process (step S17, step S223), and the poor blood collection determination is performed. FIG. 23 shows the coagulation waveform data obtained in the present embodiment. As shown in FIG. 23, the coagulation waveform data is one-dimensional array data in which transmitted-light intensities obtained by the controller 24 are chronologically arrayed in the order in which the transmitted-light intensities are obtained. The coagulation waveform data may be two-dimensional array data (see FIG. 10A) indicating the transmitted-light intensities and a time at which each transmitted-light intensity is obtained, similarly to the coagulation waveform data of the first embodiment.

A learning method for the deep learning algorithm according to the present embodiment will be described with reference to FIG. 24. A deep learning algorithm 50 is not limited to any specific one as long as the deep learning algorithm 50 is an algorithm having a neural network structure, and may be, for example, a convolutional neural network, a full connect neural network, or a combination thereof. As training data for learning of the deep learning algorithm 50, the coagulation waveform data obtained from a specimen determined as a poorly collected blood specimen by the experienced expert, and the coagulation waveform data obtained from a specimen that is collected from the same donor as the donor of the specimen determined as the poorly collected blood specimen and is determined as a normal specimen by the experienced expert, are used. To the coagulation waveform data obtained from the specimen determined as the poorly collected blood specimen, a label indicating a poorly collected blood specimen is assigned. To the coagulation waveform data obtained from the specimen determined as the normal specimen, a label indicating a normal specimen is assigned. The coagulation waveform data to which such a label is assigned is prepared from specimens of a plurality of donors, and a plurality (for example, 100 specimens or more including both the poorly collected blood specimens and the normal specimens) of pieces of the coagulation waveform data and each label assigned to the coagulation waveform data are inputted to the deep learning algorithm 50.

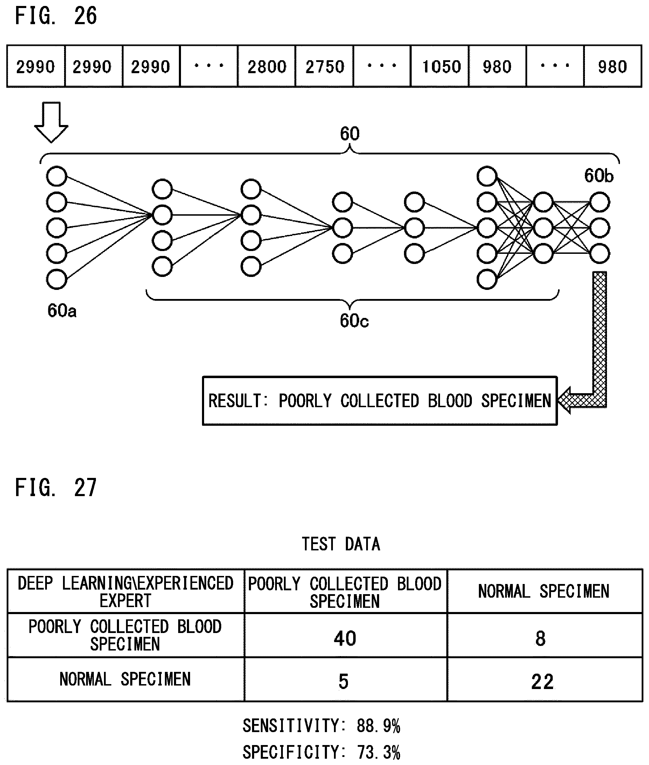

The deep learning algorithm 50 includes an input layer 50a, an output layer 50b, and a middle layer 50c formed of a plurality of layers. The coagulation waveform data is inputted to the input layer 50a, and the label assigned to the coagulation waveform data is inputted to the output layer 50b. FIG. 24 shows an example in which the label indicating a poorly collected blood specimen is inputted to the output layer 50b. Through these inputs, a weight of each layer in the middle layer 50c is calculated, and a learned deep learning algorithm 60 (FIG. 26) is generated. The learned deep learning algorithm 60 is stored in the storage unit 13.

FIG. 25 is a flow chart showing the poor blood collection determination process of the present embodiment. In step S2001, the controller 10 or the controller 30 operates to read out the coagulation waveform data stored in the storage unit 13, and input the coagulation waveform data having been read out, to the deep learning algorithm 60. The controller 10 or the controller 30 determines whether or not blood collection is poor, based on the label outputted from the deep learning algorithm 60, in step S2002.

FIG. 26 schematically shows process steps of steps S2001 and S2002. The deep learning algorithm 60 includes an input layer 60a, an output layer 60b, and a middle layer 60c formed of a plurality of layers, similarly to the deep learning algorithm 50. The coagulation waveform data obtained from a specimen for which determination as to poor blood collection is to be performed is inputted to the input layer 60a. The deep learning algorithm 60 outputs a label indicating a poorly collected blood specimen or a label indicating a normal specimen from the output layer 60b based on a probability of a poorly collected blood specimen or a normal specimen according to the result of the process performed in the middle layer 60c. FIG. 26 shows an example in which a label indicating a poorly collected blood specimen is outputted. The controller 10 or the controller 30 determines the specimen as a poorly collected blood specimen in step S2002 when the label indicating a poorly collected blood specimen is outputted from the output layer 60b, and determines the specimen as a normal specimen in step S2002 when the label indicating a normal specimen is outputted. The output layer 60b may output a probability of a poorly collected blood specimen or a probability of a normal specimen instead of the label. In this case, the controller 10 or the controller 30 may set a determination result based on a higher one of the probability of a poorly collected blood specimen and the probability of a normal specimen, or set the probability outputted from the output layer 60b as the determination result.

### (Example 4)

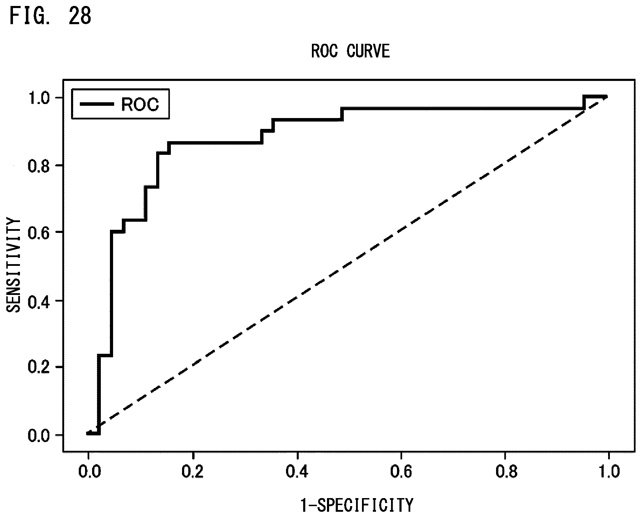

FIGS. 27 and 28 show an example of the fourth embodiment. The experienced expert of the test selected poorly collected blood specimens and normal specimens according to the criteria and a selection method for determining poor blood collection in the site of the clinical laboratory test as described above, to prepare 250 specimens, in total, including 126 poorly collected blood specimens and 124 normal specimens. Among them, coagulation waveform data of 175 specimens, in total, including 81 poorly collected blood specimens and 94 normal specimens was used as data for learning, and learning of the deep learning algorithm 50 was performed. The coagulation waveform data of the remaining specimens was inputted to the deep learning algorithm 60 obtained by the learning, and determination results of poor blood collection were obtained. The remaining specimens were 75 specimens, in total, including 45 poorly collected blood specimens and 30 normal specimens. The deep learning algorithm 60 was set so as to output a label indicating a poorly collected blood specimen when a probability of a poorly collected blood specimen which was outputted from the output layer 60b was 0.50 (50%) or more, and output a label indicating a normal specimen when a probability of a poorly collected blood specimen was less than 0.50 (50%). That is, the cutoff for the probability for determining a poorly collected blood specimen was set as 0.50.

In FIG. 27, the poorly collected blood specimens in the second column represent poorly collected blood specimens selected by the experienced expert, and the number of the specimens was 45 (=40+5). The normal specimens in the third column represent normal specimens selected by the experienced expert, and the number of the specimens was 30 (=8+22). The poorly collected blood specimens in the second line represent poorly collected blood specimens determined by the poor blood collection determination process using the deep learning algorithm, and the number of the specimens was 48 (=40+8). The normal specimens in the third line represent normal specimens determined by the poor blood collection determination process using the deep learning algorithm, and the number of the specimens was 27 (=5+22). A ratio of the number of the specimens determined as poorly collected blood specimens by both the experienced expert and the deep learning algorithm, relative to the number of the specimens determined as poorly collected blood specimens by the experienced expert represents a sensitivity, and the sensitivity was 88.9% (=40/45×100) in this example. A ratio of the number of specimens determined as normal specimens by both the experienced expert and the deep learning algorithm, relative to the number of specimens determined as normal specimens by the experienced expert represents a specificity, and the specificity was 73.3% (=22/30×100) in this example. The sensitivity and the specificity indicated high values, and fractionation performance was confirmed to be high also in this modification.

FIG. 28 shows an ROC curve indicating a sensitivity and a specificity in a case where the cutoff for a probability for determining a poorly collected blood specimen is changed. The AUC obtained from the ROC curve was 0.876, and this also indicates that fractionation performance was high in the modification.

## Claims

1. A poor blood collection determination method comprising:
mixing a blood specimen and a blood coagulation measurement reagent to prepare a measurement sample;
obtaining measurement values representing a degree of coagulation of the blood specimen from the measurement sample;
obtaining coagulation waveform data of the blood specimen based on a plurality of the measurement values; and
performing determination as to poor blood collection of the blood specimen based on the coagulation waveform data,
**characterised in that**
the performing the determination comprises obtaining values of a plurality of kinds of parameters concerning coagulation of the blood specimen based on the coagulation waveform data, and performing determination as to poor blood collection of the blood specimen based on the obtained values of the plurality of kinds of parameters,
wherein the plurality of kinds of parameters include one or more kinds of parameters concerning a coagulation rate of a blood specimen and one or more kinds of parameters concerning a coagulation time of a blood specimen.

2. The poor blood collection determination method according to claim 1, wherein a value of at least one of the plurality of kinds of parameters is determined based on a differential coagulation waveform of a coagulation waveform that is based on the coagulation waveform data.

3. The poor blood collection determination method according to claim 1 or 2, wherein the values of the plurality of kinds of parameters are determined based on a predetermined mathematical model fitted to a coagulation waveform that is based on the coagulation waveform data.

4. The poor blood collection determination method according to any one of claims 1 to 3, wherein the parameter concerning a coagulation time of the blood specimen represents a time from a time when a blood coagulation starting reagent is added to the blood specimen or a time when a coagulation reaction of the blood specimen starts, to a time when the blood specimen reaches a predetermined coagulation state.

5. The poor blood collection determination method according to any one of claims 1 to 3, wherein the parameter concerning a coagulation rate of the blood specimen represents a coagulation rate obtained when a coagulation rate of the blood specimen is a maximum rate, a maximum coagulation acceleration or a maximum coagulation deceleration.

6. The poor blood collection determination method according to any one of claims 1 to 5, wherein the blood coagulation measurement reagent is a reagent for measuring an activated partial thromboplastin time.

7. The poor blood collection determination method according to any one of claims 1 to 6, wherein, when a result of the determination indicates that blood collection of the blood specimen is poor, information indicating the result of the determination is presented.

8. A blood coagulation analysis method comprising:
steps of the poor blood collection determination method of any one of claims 1 to 7; and
obtaining an analytic value concerning coagulability of the blood specimen based on the coagulation waveform data.

9. The blood coagulation analysis method of claim 8, wherein the analytic value represents a time from a time when a blood coagulation starting reagent is added to the blood specimen or a time when a coagulation reaction of the blood specimen starts, to a time when the blood specimen reaches a predetermined coagulation state.

10. The blood coagulation analysis method of claim 8 or 9, wherein, when a result of the determination indicates that blood collection of the blood specimen is poor, the analytic value is prohibited from being presented.

11. A method performed by a computer for performing determination as to poor blood collection of a blood specimen, the method comprising:
receiving coagulation waveform data, of a blood specimen, which is based on measurement values that represent a degree of coagulation of the blood specimen and that are obtained from a measurement sample containing the blood specimen and a blood coagulation measurement reagent; and
performing determination as to poor blood collection of the blood specimen based on the coagulation waveform data,
**characterised in that**
values of a plurality of kinds of parameters concerning coagulation of a blood specimen are obtained on the basis of coagulation waveform data based on the measurement values representing a degree of coagulation, and determination as to poor blood collection of the blood specimen is performed based on the obtained values of the plurality of kinds of parameters,
wherein the plurality of kinds of parameters include one or more kinds of parameters concerning a coagulation rate of a blood specimen and one or more kinds of parameters concerning a coagulation time of a blood specimen.

12. The method of claim 11 for performing determination as to poor blood collection of a blood specimen, wherein the receiving the coagulation waveform data comprises receiving the coagulation waveform data of the blood specimen via a network from a device which generates the coagulation waveform data based on a measurement unit for obtaining the measurement value.

13. A method performed by a computer for performing determination as to poor blood collection of a blood specimen, the method **characterised by** comprising:
receiving values of a plurality of kinds of parameters concerning coagulation of a blood specimen, the values of the plurality of kinds of parameters being obtained from coagulation waveform data, of the blood specimen, which is based on measurement values that represent a degree of coagulation of the blood specimen and that are obtained from a measurement sample containing the blood specimen and a blood coagulation measurement reagent; and
performing determination as to poor blood collection of the blood specimen based on the values of the plurality of kinds of parameters,
wherein the plurality of kinds of parameters include one or more kinds of parameters concerning a coagulation rate of a blood specimen and one or more kinds of parameters concerning a coagulation time of a blood specimen.

14. A blood coagulation analysis system (1000, 2000) comprising:
a sample preparation unit (23) configured to mix a blood specimen and a blood coagulation measurement reagent to prepare a measurement sample;
a detector (22) configured to obtain measurement values representing a degree of coagulation of the blood specimen, from the measurement sample; and
a controller (10, 24, 101, 102) configured to generate coagulation waveform data of the blood specimen based on the measurement values, obtain an analytic value concerning coagulability of the blood specimen based on the coagulation waveform data, and perform determination as to poor blood collection of the blood specimen based on the coagulation waveform data,
**characterised in that**
values of a plurality of kinds of parameters concerning coagulation of a blood specimen are obtained on the basis of coagulation waveform data based on the measurement values representing a degree of coagulation, and determination as to poor blood collection of the blood specimen is performed based on the obtained values of the plurality of kinds of parameters,
wherein the plurality of kinds of parameters include one or more kinds of parameters concerning a coagulation rate of a blood specimen and one or more kinds of parameters concerning a coagulation time of a blood specimen.

## Patentansprüche

1. Verfahren zur Bestimmung einer unzureichenden Blutentnahme, umfassend:
Mischen einer Blutprobe und eines Blutgerinnungsmessreagenzes, um eine Messprobe herzustellen;
Erhalten von Messwerten, die einen Gerinnungsgrad der Blutprobe repräsentieren, von der Messprobe;
Erhalten von Gerinnungswellenformdaten der Blutprobe basierend auf einer Vielzahl der Messwerte; und
Durchführen einer Bestimmung hinsichtlich einer unzureichenden Blutentnahme der Blutprobe basierend auf den Gerinnungswellenformdaten,
**dadurch gekennzeichnet, dass**
das Durchführen der Bestimmung das Erhalten von Werten einer Vielzahl von Arten von Parametern betreffend die Gerinnung der Blutprobe basierend auf den Gerinnungswellenformdaten und das Durchführen einer Bestimmung hinsichtlich einer unzureichenden Blutentnahme der Blutprobe basierend auf den erhaltenen Werten der Vielzahl von Arten von Parametern umfasst,
wobei die Vielzahl von Arten von Parametern eine oder mehrere Arten von Parametern betreffend eine Gerinnungsrate einer Blutprobe und eine oder mehrere Arten von Parametern betreffend eine Gerinnungszeit einer Blutprobe einschließt.

2. Verfahren zur Bestimmung einer unzureichenden Blutentnahme nach Anspruch 1, wobei ein Wert von mindestens einer der Vielzahl von Arten von Parametern basierend auf einer differentiellen Gerinnungswellenform einer Gerinnungswellenform bestimmt wird, die auf den Gerinnungswellenformdaten basiert.

3. Verfahren zur Bestimmung einer unzureichenden Blutentnahme nach Anspruch 1 oder 2, wobei die Werte der Vielzahl von Arten von Parametern basierend auf einem vorbestimmten mathematischen Modell bestimmt werden, das an eine Gerinnungswellenform angepasst ist, die auf den Gerinnungswellenformdaten basiert.

4. Verfahren zur Bestimmung einer unzureichenden Blutentnahme nach einem der Ansprüche 1 bis 3, wobei der Parameter betreffend eine Gerinnungszeit der Blutprobe eine Zeit von einem Zeitpunkt, zu dem ein Blutgerinnungs-Startreagenz zu der Blutprobe zugegeben wird, oder einem Zeitpunkt, zu dem eine Gerinnungsreaktion der Blutprobe startet, bis zu einem Zeitpunkt, zu dem die Blutprobe einen vorbestimmten Gerinnungszustand erreicht, repräsentiert.

5. Verfahren zur Bestimmung einer unzureichenden Blutentnahme nach einem der Ansprüche 1 bis 3, wobei der Parameter betreffend eine Gerinnungsrate der Blutprobe eine Gerinnungsrate repräsentiert, die erhalten wird, wenn eine Gerinnungsrate der Blutprobe eine maximale Rate, eine maximale Gerinnungsbeschleunigung oder eine maximale Gerinnungsverzögerung ist.

6. Verfahren zur Bestimmung einer unzureichenden Blutentnahme nach einem der Ansprüche 1 bis 5, wobei das Blutgerinnungsmessreagenz ein Reagenz zum Messen einer aktivierten partiellen Thromboplastinzeit ist.

7. Verfahren zur Bestimmung einer unzureichenden Blutentnahme nach einem der Ansprüche 1 bis 6, wobei, wenn ein Ergebnis der Bestimmung anzeigt, dass eine Blutentnahme der Blutprobe unzureichend ist, Informationen präsentiert werden, die das Ergebnis der Bestimmung anzeigen.

8. Blutgerinnungsanalyseverfahren, umfassend:
Schritte des Verfahrens zur Bestimmung einer unzureichenden Blutentnahme nach einem der Ansprüche 1 bis 7; und
Erhalten eines analytischen Wertes betreffend die Gerinnbarkeit der Blutprobe basierend auf den Gerinnungswellenformdaten.

9. Blutgerinnungsanalyseverfahren nach Anspruch 8, wobei der analytische Wert eine Zeit von einem Zeitpunkt, zu dem ein Blutgerinnungs-Startreagenz zu der Blutprobe zugegeben wird, oder einem Zeitpunkt, zu dem eine Gerinnungsreaktion der Blutprobe startet, bis zu einem Zeitpunkt, zu dem die Blutprobe einen vorbestimmten Gerinnungszustand erreicht, repräsentiert.

10. Blutgerinnungsanalyseverfahren nach Anspruch 8 oder 9, wobei, wenn ein Ergebnis der Bestimmung anzeigt, dass eine Blutentnahme der Blutprobe unzureichend ist, verhindert wird, dass der analytische Wert präsentiert wird.

11. Computerimplementiertes Verfahren zur Durchführung einer Bestimmung hinsichtlich einer unzureichenden Blutentnahme einer Blutprobe, wobei das Verfahren umfasst:
Empfangen von Gerinnungswellenformdaten einer Blutprobe, die auf Messwerten basieren, die einen Gerinnungsgrad der Blutprobe repräsentieren und die aus einer Messprobe erhalten werden, die die Blutprobe und ein Blutgerinnungsmessreagenz enthält; und
Durchführen einer Bestimmung hinsichtlich einer unzureichenden Blutentnahme der Blutprobe basierend auf den Gerinnungswellenformdaten,
**dadurch gekennzeichnet, dass**
Werte einer Vielzahl von Arten von Parametern betreffend die Gerinnung einer Blutprobe auf der Basis von Gerinnungswellenformdaten basierend auf den Messwerten, die einen Gerinnungsgrad repräsentieren, erhalten werden, und eine Bestimmung hinsichtlich einer unzureichenden Blutentnahme der Blutprobe basierend auf den erhaltenen Werten der Vielzahl von Arten von Parametern durchgeführt wird,
wobei die Vielzahl von Arten von Parametern eine oder mehrere Arten von Parametern betreffend eine Gerinnungsrate einer Blutprobe und eine oder mehrere Arten von Parametern betreffend eine Gerinnungszeit einer Blutprobe einschließt.

12. Verfahren nach Anspruch 11 zur Durchführung einer Bestimmung hinsichtlich einer unzureichenden Blutentnahme einer Blutprobe, wobei das Empfangen der Gerinnungswellenformdaten das Empfangen der Gerinnungswellenformdaten der Blutprobe über ein Netzwerk von einer Vorrichtung umfasst, die die Gerinnungswellenformdaten basierend auf einer Messeinheit zum Erhalten des Messwerts erzeugt.

13. Computerimplementiertes Verfahren zur Durchführung einer Bestimmung hinsichtlich einer unzureichenden Blutentnahme einer Blutprobe, wobei das Verfahren **gekennzeichnet ist durch**
das Empfangen von Werten einer Vielzahl von Arten von Parametern betreffend die Gerinnung einer Blutprobe, wobei die Werte der Vielzahl von Arten von Parametern aus Gerinnungswellenformdaten der Blutprobe erhalten werden, die auf Messwerten basieren, die einen Gerinnungsgrad der Blutprobe repräsentieren und die aus einer Messprobe erhalten werden, die die Blutprobe und ein Blutgerinnungsmessreagenz enthält; und
das Durchführen einer Bestimmung hinsichtlich einer unzureichenden Blutentnahme der Blutprobe basierend auf den Werten der Vielzahl von Arten von Parametern,
wobei die Vielzahl von Arten von Parametern eine oder mehrere Arten von Parametern betreffend eine Gerinnungsrate einer Blutprobe und eine oder mehrere Arten von Parametern betreffend eine Gerinnungszeit einer Blutprobe einschließt.

14. Blutgerinnungsanalysesystem (1000, 2000), umfassend:
eine Probenpräparationseinheit (23), die konfiguriert ist, um eine Blutprobe und ein Blutgerinnungsmessreagenz zu mischen, um eine Messprobe herzustellen;
einen Detektor (22), der konfiguriert ist, um Messwerte, die einen Gerinnungsgrad der Blutprobe repräsentieren, von der Messprobe zu erhalten; und
eine Steuerung (10, 24, 101, 102), die konfiguriert ist, um Gerinnungswellenformdaten der Blutprobe basierend auf den Messwerten zu erzeugen, einen analytischen Wert betreffend die Gerinnbarkeit der Blutprobe basierend auf den Gerinnungswellenformdaten zu erhalten, und eine Bestimmung hinsichtlich einer unzureichenden Blutentnahme der Blutprobe basierend auf den Gerinnungswellenformdaten durchzuführen, **dadurch gekennzeichnet, dass**
Werte einer Vielzahl von Arten von Parametern betreffend die Gerinnung einer Blutprobe auf der Basis von Gerinnungswellenformdaten basierend auf den Messwerten, die einen Gerinnungsgrad repräsentieren, erhalten werden, und eine Bestimmung hinsichtlich einer unzureichenden Blutentnahme der Blutprobe basierend auf den erhaltenen Werten der Vielzahl von Arten von Parametern durchgeführt wird,
wobei die Vielzahl von Arten von Parametern eine oder mehrere Arten von Parametern betreffend eine Gerinnungsrate einer Blutprobe und eine oder mehrere Arten von Parametern betreffend eine Gerinnungszeit einer Blutprobe einschließt.

## Revendications

1. Procédé de détermination d'un prélèvement sanguin défectueux comprenant :
le mélange d'un spécimen de sang et d'un réactif de mesure de coagulation sanguine pour préparer un échantillon de mesure ;
l'obtention de valeurs de mesure représentant un degré de coagulation du spécimen de sang à partir de l'échantillon de mesure ;
l'obtention de données de forme d'onde de coagulation du spécimen de sang sur la base d'une pluralité des valeurs de mesure ; et
la réalisation d'une détermination quant à un prélèvement sanguin défectueux du spécimen de sang sur la base des données de forme d'onde de coagulation,
**caractérisé en ce que**
la réalisation de la détermination comprend l'obtention de valeurs d'une pluralité de types de paramètres concernant la coagulation du spécimen de sang sur la base des données de forme d'onde de coagulation, et la réalisation d'une détermination quant à un prélèvement sanguin défectueux du spécimen de sang sur la base des valeurs obtenues de la pluralité de types de paramètres,
dans lequel la pluralité de types de paramètres comprend un ou plusieurs types de paramètres concernant une vitesse de coagulation d'un spécimen de sang et un ou plusieurs types de paramètres concernant un temps de coagulation d'un spécimen de sang.

2. Procédé de détermination d'un prélèvement sanguin défectueux selon la revendication 1, dans lequel une valeur d'au moins l'un de la pluralité de types de paramètres est déterminée sur la base d'une forme d'onde de coagulation différentielle d'une forme d'onde de coagulation qui est basée sur les données de forme d'onde de coagulation.

3. Procédé de détermination d'un prélèvement sanguin défectueux selon la revendication 1 ou 2, dans lequel les valeurs de la pluralité de types de paramètres sont déterminées sur la base d'un modèle mathématique prédéterminé ajusté à une forme d'onde de coagulation qui est basée sur les données de forme d'onde de coagulation.

4. Procédé de détermination d'un prélèvement sanguin défectueux selon l'une quelconque des revendications 1 à 3, dans lequel le paramètre concernant un temps de coagulation du spécimen de sang représente un temps à partir d'un moment où un réactif de démarrage de coagulation sanguine est ajouté au spécimen de sang ou d'un moment où une réaction de coagulation du spécimen de sang démarre, jusqu'à un moment où le spécimen de sang atteint un état de coagulation prédéterminé.

5. Procédé de détermination d'un prélèvement sanguin défectueux selon l'une quelconque des revendications 1 à 3, dans lequel le paramètre concernant une vitesse de coagulation du spécimen de sang représente une vitesse de coagulation obtenue lorsqu'une vitesse de coagulation du spécimen de sang est une vitesse maximale, une accélération de coagulation maximale ou une décélération de coagulation maximale.

6. Procédé de détermination d'un prélèvement sanguin défectueux selon l'une quelconque des revendications 1 à 5, dans lequel le réactif de mesure de coagulation sanguine est un réactif pour mesurer un temps de thromboplastine partielle activée.

7. Procédé de détermination d'un prélèvement sanguin défectueux selon l'une quelconque des revendications 1 à 6, dans lequel, lorsqu'un résultat de la détermination indique que le prélèvement sanguin du spécimen de sang est défectueux, des informations indiquant le résultat de la détermination sont présentées.

8. Procédé d'analyse de la coagulation sanguine comprenant :
des étapes du procédé de détermination d'un prélèvement sanguin défectueux selon l'une quelconque des revendications 1 à 7 ; et
l'obtention d'une valeur analytique concernant la coagulabilité du spécimen de sang sur la base des données de forme d'onde de coagulation.

9. Procédé d'analyse de la coagulation sanguine selon la revendication 8, dans lequel la valeur analytique représente un temps à partir d'un moment où un réactif de démarrage de coagulation sanguine est ajouté au spécimen de sang ou d'un moment où une réaction de coagulation du spécimen de sang démarre, jusqu'à un moment où le spécimen de sang atteint un état de coagulation prédéterminé.

10. Procédé d'analyse de la coagulation sanguine selon la revendication 8 ou 9, dans lequel, lorsqu'un résultat de la détermination indique que le prélèvement sanguin du spécimen de sang est défectueux, la présentation de la valeur analytique est interdite.

11. Procédé exécuté par un ordinateur pour réaliser une détermination quant à un prélèvement sanguin défectueux d'un spécimen de sang, le procédé comprenant :
la réception de données de forme d'onde de coagulation, d'un spécimen de sang, qui sont basées sur des valeurs de mesure qui représentent un degré de coagulation du spécimen de sang et qui sont obtenues à partir d'un échantillon de mesure contenant le spécimen de sang et un réactif de mesure de coagulation sanguine ; et
la réalisation d'une détermination quant à un prélèvement sanguin défectueux du spécimen de sang sur la base des données de forme d'onde de coagulation,
**caractérisé en ce que**
des valeurs d'une pluralité de types de paramètres concernant la coagulation d'un spécimen de sang sont obtenues sur la base de données de forme d'onde de coagulation basées sur les valeurs de mesure représentant un degré de coagulation, et une détermination quant à un prélèvement sanguin défectueux du spécimen de sang est réalisée sur la base des valeurs obtenues de la pluralité de types de paramètres,
dans lequel la pluralité de types de paramètres comprend un ou plusieurs types de paramètres concernant une vitesse de coagulation d'un spécimen de sang et un ou plusieurs types de paramètres concernant un temps de coagulation d'un spécimen de sang.

12. Procédé selon la revendication 11 pour réaliser une détermination quant à un prélèvement sanguin défectueux d'un spécimen de sang, dans lequel la réception des données de forme d'onde de coagulation comprend la réception des données de forme d'onde de coagulation du spécimen de sang via un réseau à partir d'un dispositif qui génère les données de forme d'onde de coagulation sur la base d'une unité de mesure pour obtenir la valeur de mesure.

13. Procédé exécuté par un ordinateur pour réaliser une détermination quant à un prélèvement sanguin défectueux d'un spécimen de sang, le procédé **caractérisé par**
la réception de valeurs d'une pluralité de types de paramètres concernant la coagulation d'un spécimen de sang, les valeurs de la pluralité de types de paramètres étant obtenues à partir de données de forme d'onde de coagulation, du spécimen de sang, qui sont basées sur des valeurs de mesure qui représentent un degré de coagulation du spécimen de sang et qui sont obtenues à partir d'un échantillon de mesure contenant le spécimen de sang et un réactif de mesure de coagulation sanguine ; et
la réalisation d'une détermination quant à un prélèvement sanguin défectueux du spécimen de sang sur la base des valeurs de la pluralité de types de paramètres,
dans lequel la pluralité de types de paramètres comprend un ou plusieurs types de paramètres concernant une vitesse de coagulation d'un spécimen de sang et un ou plusieurs types de paramètres concernant un temps de coagulation d'un spécimen de sang.

14. Système d'analyse de la coagulation sanguine (1000, 2000) comprenant :
une unité de préparation d'échantillon (23) configurée pour mélanger un spécimen de sang et un réactif de mesure de coagulation sanguine pour préparer un échantillon de mesure ;
un détecteur (22) configuré pour obtenir des valeurs de mesure représentant un degré de coagulation du spécimen de sang, à partir de l'échantillon de mesure ; et
un contrôleur (10, 24, 101, 102) configuré pour générer des données de forme d'onde de coagulation du spécimen de sang sur la base des valeurs de mesure, obtenir une valeur analytique concernant la coagulabilité du spécimen de sang sur la base des données de forme d'onde de coagulation, et réaliser une détermination quant à un prélèvement sanguin défectueux du spécimen de sang sur la base des données de forme d'onde de coagulation,
**caractérisé en ce que**
des valeurs d'une pluralité de types de paramètres concernant la coagulation d'un spécimen de sang sont obtenues sur la base de données de forme d'onde de coagulation basées sur les valeurs de mesure représentant un degré de coagulation, et une détermination quant à un prélèvement sanguin défectueux du spécimen de sang est réalisée sur la base des valeurs obtenues de la pluralité de types de paramètres,
dans lequel la pluralité de types de paramètres comprend un ou plusieurs types de paramètres concernant une vitesse de coagulation d'un spécimen de sang et un ou plusieurs types de paramètres concernant un temps de coagulation d'un spécimen de sang.
